# EUROPEAN PATENT APPLICATION

(11) **EP 4 066 629 A1**
(43) Date of publication of application: **05.10.2022**
(21) Application number: 21305430.7
(22) Date of filing: 02.04.2021
(51) Int. Cl.: A01H 1/00, A01H 3/04, C07K 7/08, A01N 33/00

(54) **METHODS FOR ALTERING THE MICROBIOME INFLUENCED BY ROOTS OF A PLANT USING MICROPEPTIDES**

(71) Applicant: Université de Rennes 1, 35065 Rennes Cedex (FR); Centre national de la recherche scientifique, 75016 Paris (FR); UNIVERSITE PAUL SABATIER TOULOUSE III, 31062 Toulouse Cedex 9 (FR); Institut National De La Recherche Scientifique (INRS), Québec, QC G1K 9A9 (CA)
(72) Inventor: EL AMRANI, Abdelhak, 35630 SAINT SYMPHORIEN (FR); MIDDLETON, Harriet, 35510 CESSON-SEVIGNE (FR); MONARD, Cécile, 35200 RENNES (FR); COMBIER, Jean-Philippe, 31320 AUZEVILLE TOLOSANE (FR); YERGEAU, Etienne, MONTRÉAL, Québec H4L 3B8 (CA)
(74) Representative: Lavoix

(57) **Abstract**

The present invention relates to a method for selecting at least one micropeptide able to alter the microbiome influenced by roots of a plant based on identifying miRNAs secreted by said plant in a sample of the roots and/or rhizosphere.

The present invention also relates to a micropeptide able to alter the microbiome influenced by roots of a plant and its use as a plant microbiome modulating agent, for example for inducing a phenotype of interest, such as a pathogen resistance and/or a water resistance.

## Description

### Field of the invention

The present invention concerns a method for altering the microbiome influenced by a plant's root.

### Technical background

Plant-associated microorganisms are essential for the well-being of plants. It is believed that plants and their associated microorganisms communicate in both directions, that is to say from the plants to microorganisms and from the microorganisms to the plants. Plants particularly interact with their associated microorganisms via rhizodeposits, which are compounds released by the roots into their environment.

It was recently hypothesized that plants and microorganisms could interact using extracellular vesicles containing small RNA (sRNA) (see Middleton et al., 2021, Trends Plant Sci., 26(2):132-141).

Rhizosphere engineering thus represents in interesting approach to favor the development of plants by modulating their rhizomicrobiome.

Rhizosphere engineering is currently carried out by (i) adding molecules of the primary or secondary metabolism, to favor the development of the microbiome or (ii) inoculating bacterial or fungal strains selected on the basis of their positive effect on the plant. However, both approaches have shown drawbacks.

Using molecules of the primary or secondary metabolism indeed does not allow promoting the development of specific strains. Consequently, this technique may result in the development of beneficial microorganisms, but also of microorganisms having a negative effect on the plant.

Besides, inoculating bacterial or fungal strains present two majors issues: either the strain becomes invasive and destabilizes the whole microbiome, either the strain is not capable to maintain itself at a sufficient level in a microbiome showing high competition.

There is therefore a need of methods allowing altering the microbiome influenced by roots of a plant, preferably in a targeted manner, for example by promoting or inhibiting the development of specific microorganisms.

### Description

The Inventors have found that some miRNAs expressed by a plant cell are secreted in the rhizosphere, wherein they directly acts on specific bacteria or fungi present by gene silencing or activation. A plant is thus capable of modifying the microbiome of its rhizosphere by secreting specific miRNAs, which for example result in an increased or decreased population of specific microorganisms. These miRNAs are referred to as secreted miRNAs in the following.

The Inventors have then unexpectedly shown that it is possible to alter the rhizomicrobiome of a plant, by directly watering said plant with a composition comprising a micropeptide able to induce the transcription of a specific secreted miRNA.

By the term "rhizomicrobiome", it is herein meant the microbiome of a rhizosphere.

The claimed method for altering the microbiome influenced by roots of a plant thus advantageously allows specifically targeting one or several strains of microorganisms, to activate or inhibit their development, without using a GMO approach.

Besides, the claimed method for altering the microbiome influenced by roots of a plant is easy of use, since it only required watering the plant with a composition comprising a suitable micropeptide.

The micropeptide can be produced easily, in high quantity and at low costs, by recombinant technology.

Interestingly, the method for altering the microbiome influenced by roots of a plant can be used to induce a phenotype of interest of the plant. For example, the micropeptide mi159c of sequence SEQ ID NO: 3 allows conferring drought resistance to Arabidopsis thaliana.

A first object of the invention is a method for selecting at least one micropeptide able to alter the microbiome influenced by roots of a plant, wherein said method comprises:
a) identifying (i) at least one miRNA secreted by said plant in a sample of the roots and/or rhizosphere of said plant or (ii) at least one miRNA secreted by said plant which is identical to a secreted miRNA of a control plant, wherein the secreted miRNA of the control plant is able to alter the microbiome influenced by roots of said control plant,
b) identifying at least one ORF associated to at least one miRNA identified in step a) and coding for a micropeptide,
c) providing at least one micropeptide encoded by one ORF identified in step b) and applying said micropeptide onto said plant,
d) comparing the microbiome influenced by roots of the plant in the presence or absence of said micropeptide, and
e) selecting at least one micropeptide able to alter the microbiome influenced by roots of the plant.

The micropeptide in step c) is preferably obtained by chemical synthesis or by recombinant technology.

Step d) preferably comprises determining the genus and/or species of microorganisms and, optionally, their proportion, quantity and/or concentration, in a sample of the roots and/or rhizosphere of the plant in the presence of said micropeptide and in a sample of the roots and/or rhizosphere of the plant absence of said micropeptide.

Another object of the invention is a micropeptide, wherein said micropeptide is able to alter the microbiome influenced by roots of a plant.

Said micropeptide is preferably selected by the method as defined above.

Said micropeptide preferably comprises a sequence selected from the group consisting of sequence SEQ ID NO: 1, SEQ ID NO: 2 and SEQ ID NO: 3.

Another object of the invention is a composition comprising at least one micropeptide as defined above.

Another object of the invention is a device suitable for spraying or watering, wherein said device comprising at least one micropeptide as defined above or a composition as defined above.

Another objet of the invention is a method for altering the microbiome influenced by roots of a plant, comprising administering to said plant at least one micropeptide as defined above. Said micropeptide is preferably provided in the form of a composition as defined above or in a device as defined above. In said method, altering the microbiome of the plant preferably induces a phenotype of interest in the plant.

Another object of the invention is the use of a micropeptide as defined above as a plant microbiome modulating agent, in particular to induce a phenotype of interest in a plant. Said phenotype of interest is preferably selected from the group consisting of a resistance to at least one plant pathogen, a resistance to at least one stress, or a resistance to at least one pollutant and their combinations. Said micropeptide is preferably provided in the form of a composition as defined above or in a device as defined above.

### Plant

The plant is any plant having roots and being able to grow in a soil or substrate.

The plant as defined above is preferably a flowering plant.

The plant as defined above is preferably a monocotyledon or a dicotyledon.

The plant as defined above may be a plant grown in a greenhouse or in crop.

The plant as defined above is preferably an agronomic plant.

By the term "agronomic plant", it is herein meant a plant, which is produced on a large scale, in particular for human and animal food or for industrial purposes, such as biofuel.

The agronomic plant as defined above may for example be:
- a cereal, such as wheat, rice, corn, barley, rye, oat, sorghum or millet,
- a legume, such as beans, broad beans, peas, chickpeas, cow peas, lentils or lupin,
- an oilseed, such as soybean, sunflower or canola,
- a fiber crop, such as cotton,
- A sugar crop, such as sugar beet or sugar cane, or
- A starchy root or tuber crop, such as potato, sweet potato, yam or taro.

### Micropeptide

The present invention particularly relates to a micropeptide able to alter the microbiome influenced by roots of a plant, preferably able to alter the rhizomicrobiome.

The micropeptide as defined above indeed induces the transcription of a secreted miRNA, i.e. a miRNA which is secreted by the plant cells in the rhizosphere. Said secreted miRNA is then able to alter the microbiome influenced by roots of a plant. The micropeptide of the invention is thus able to alter the microbiome influenced by roots of a plant, via the induced transcription of a specific secreted miRNA.

By the expression "microbiome influenced by roots of a plant", it is herein meant microorganisms living in the rhizosphere and/or the phyllosphere and/or the endosphere of said plant and which receives molecules from said plant. The microbiome influenced by roots of a plant is preferably the rhizomicrobiome, i.e. the microorganisms living in the rhizosphere.

By the expression "able to alter the microbiome influenced by roots of a plant", it is herein meant that the micropeptide is able to induce at least one change in the microbiome influenced by roots, such as for example an increase or decrease in the proportion, quantity and/or concentration of at least one microorganism.

Said microorganism may for example be a bacterium or a fungus.

The bacterium may for example be known for its capacity to increase plant growth, resistance to pathogens, tolerance to environmental stress and/or tolerance to pollutants.

The fungus may for example be known for its capacity to increase plant growth, resistance to pathogens, tolerance to environmental stress and/or tolerance to pollutants.

The skilled person can easily determine whether a given micropeptide is able to alter the microbiome influenced by roots of a given plant. For example, plants at 6-leaves stage are treated with a solution comprising the micropeptide to be assessed or with water (control condition), three times a week, during three weeks. 500 µL of the solution comprising the micropeptide at a concentration of 20 µM in water and 500 µl of water (for the control condition) are for example used. The plant is carefully watered on the soil, at the base of the stem, i.e. on the root crown. After three weeks, the microbiome influenced by roots in the presence of the micropeptide is assessed and compared to the microbiome influenced by roots in the absence of said micropeptide.

The microbiome influenced by roots may be assessed by measuring an alpha-diversity index, such as Observed Species, Shannon or Simpson index and/or by quantifying bacteria and fungi, for example by qPCR using 16S rRNA for bacteria and/or using ITS2 for fungi.

A change in the alpha-diversity index or in the proportion, quantity and/or concentration of at least one microorganism in the presence versus in the absence of the micropeptide indicates an alteration of the microbiome and thus that the assessed peptide is able to alter the microbiome influenced by roots of the plant.

The micropeptide as defined above preferably has a length of at most 100 amino acids, preferably at most 75 amino acids, more preferably at most 50 amino acids.

The micropeptide as defined above preferably has a length of at least 3 amino acids.

The micropeptide as defined above preferably does not comprise any chemical modification and/or non-natural amino acid, in particular for increasing its stability. For the purpose of the invention, it is indeed preferred to that the micropeptide has a short remanence when used in nature.

The micropeptide as defined above may be obtained by the method of selection as defined below.

The micropeptide may for example comprise a sequence selected from the group consisting of sequence SEQ ID NO: 1, SEQ ID NO: 2 and SEQ ID NO: 3.

### Composition comprising a micropeptide

The present invention also relates to a composition comprising at least one micropeptide as defined above.

The composition may for example comprise one micropeptide as defined above, two different micropeptides as defined above or at least two different micropeptides as defined above.

When at least two micropeptides are used, they may induce transcription of the same miRNA in the plant or of different miRNAs.

The composition as defined above may for example comprise at least 1 µM of the or each micropeptide, preferably at least 5 µM of the or each micropeptide and/or at most 20 µM of the or each micropeptide, preferably at most 15 µM of the or each micropeptide.

The composition as defined above preferably comprises an aqueous solution and at least one micropeptide as defined above. The composition as defined above preferably comprises an aqueous solution and at least two micropeptides as defined above.

The aqueous solution is preferably water.

When the micropeptide is not soluble in water, an organic solvent may be used to dilute the micropeptide, before adding water. The composition may thus comprise an organic solvent, a micropeptide and water.

### Device suitable for spraying or watering

The present invention also relates to a device suitable for spraying or watering, wherein said device comprises a micropeptide as defined above or a composition as defined above.

The device may for example be a device comprising a container and means for spraying or watering, a watering can or a sprayer.

The device may be suitable for a use in a greenhouse or in crop.

### Method for selecting at least one micropeptide able to alter the microbiome influenced bv roots of a plant

The present invention also relates to a method for selecting at least one micropeptide able to alter the microbiome influenced by roots of a plant, preferably able to alter the rhizomicrobiome of a plant.

The method as defined above particularly comprises:
a) identifying (i) at least one miRNA secreted by said plant in a sample of the roots and/or rhizosphere of said plant and/or (ii) at least one miRNA secreted by said plant which is identical to a secreted miRNA of a control plant, wherein the secreted miRNA of the control plant is able to alter the microbiome influenced by roots of said control plant,
b) identifying at least one ORF associated to at least one miRNA identified in step a) and coding for a micropeptide,
c) providing at least one micropeptide encoded by one ORF identified in step b) and applying said micropeptide onto said plant,
d) comparing the microbiome influenced by roots of the plant in the presence or absence of said micropeptide, and
e) selecting at least one micropeptide able to alter the microbiome influenced by roots of the plant.

The plant is particularly as defined above.

The Inventors have surprisingly found that a plant secrete some miRNAs, which are then found in rhizosphere. These miRNAs can then act on specific microorganisms present in the rhizosphere and/or the phyllosphere and/or the endosphere by gene silencing or activation, thereby specifically promoting, inhibiting their growth or changing the interaction between microorganisms.

### Step a)

Step a) comprises identifying (i) at least one miRNA secreted by said plant in a sample of the roots and/or rhizosphere of said plant and/or (ii) at least one miRNA secreted by said plant which is identical to a secreted miRNA of a control plant, wherein the secreted miRNA of the control plant is able to alter the microbiome influenced by roots of said control plant.

In alternative (i), step a) comprises identifying at least one miRNA secreted by said plant in a sample of the roots and/or rhizosphere of said plant.

The plant may be grown in normal conditions and/or in specific conditions.

Normal conditions are the conditions well-known by the skilled person for this plant.

Specific conditions may comprise at least one condition selected from the group consisting of a specific cycle of light / darkness, a specific temperature, a specific soil or substrate, a specific watering and the presence of at least one specific plant pathogen.

The sample is preferably removed once the plant has reached full growth of the rosette before flowering, for example, preferably after 4 weeks of growth.

Preferably, the sample is a sample of the rhizosphere. In particular, in the method for selecting at least one micropeptide able to alter the rhizomicrobiome of a plant according to the invention, the sample is a sample of the rhizosphere.

By the term "rhizosphere", it is herein meant a portion of soil or substrate surrounding the root system. The rhizosphere comprises microorganisms, which for example differ from those living on the root surface (phyllosphere) or inside plant tissues (endosphere).

A sample of the rhizosphere is thus a sample of the soil or substrate in contact with the roots of the plant and/or close to the roots of the plant.

For example, roots are harvested and the soil or substrate located at the vicinity of roots is recovered and constitutes the sample of rhizosphere. Preferably, a sample of rhizosphere comprise 1 mm of the soil in contact with the roots of the plant.

Identifying at least one secreted miRNA preferably comprises purifying the miRNAs present in the sample and sequencing said miRNAs.

Purifying the miRNAs present in the sample of roots and/or rhizosphere may comprise freezing the sample at -80°C, for example in liquid nitrogen and, optionally, storing the sample at -80°C until miRNA extraction. RNA, including miRNA, may then be extracted by any suitable method, such as QIAgen Power RNA soil kit.

Sequencing the miRNA may be carried out by any method well known by the skilled person, such as with a NextSeq 500 sequencer (Illumina) in single read (1 × 50 bp, 20 million reads).

Identifying at least one secreted miRNA preferably further comprises confirming that said miRNA originates from the plant, for example by performing sequence alignments using miRNA databases of said plant.

Identifying at least one miRNA secreted by said plant in a sample of roots and/or the rhizosphere of said plant may further comprise comparing the identified miRNA with miRNA isolated in a sample of the same soil or substrate used for growing the plant, but without any plant, to confirm that the secreted miRNA originates from the plant and not from the soil and/or from the substrate used for growth.

In alternative (ii), step a) comprises identifying at least one miRNA secreted by said plant which is identical to a secreted miRNA of a control plant, wherein the secreted miRNA of the control plant is able to alter the microbiome influenced by the roots of said control plant. Preferably, the secreted miRNA of the control plant is able to alter the rhizomicrobiome of said control plant.

In particular, in the method for selecting at least one micropeptide able to alter the rhizomicrobiome of a plant according to the invention, the secreted miRNA of the control plant is able to alter the rhizomicrobiome of said control plant.

In such case, a secreted miRNA able to alter the microbiome influenced by roots of a plant has already been identified and this plant is referred to as the control plant. The aim of alternative (ii) is then to identify a micropeptide able to induce transcription of said secreted miRNA in another plant.

For example, miRNA159 is common to at least *Arabidopsis* and *Brachipodium,* but its transcription is not induced by the same micropeptide in *Arabidopsis* and *Brachipodium.*

By "another plant", it is meant a plant of a different species and/or genus.

Alternative (ii) may thus comprise analyzing the miRNA of the plant to detect the presence of at least one miRNA identical to a secreted miRNA of the control plant.

Alternative (ii) may be performed by sequence alignment using miRNA databases.

The secreted miRNA of the control plant has preferably been identified using the alternative (i) of the method as defined above.

### Step b)

Step b) comprises identifying at least one ORF associated to at least one miRNA identified in step a) and coding for a micropeptide.

miRNA transcription is indeed induced by a micropeptide, which is specific to said miRNA.

The ORF encoding a micropeptide is located in 5' of the pre-miRNA whose transcription is induced by said micropeptide.

Several ORFs encoding different micropeptides are usually identified using the sequence of the secreted miRNA identified in step a).

The ORFs associated to at least one miRNA may be identified by performing sequence alignments using miRNA databases of the plant.

The micropeptide preferably has a length of at most 100 amino acids, preferably at most 75 amino acids, more preferably at most 50 amino acids.

The micropeptide preferably has a length of at least 3 amino acids.

### Step c)

Step c) comprises providing at least one micropeptide encoded by one ORF identified in step b) and applying said micropeptide onto said plant.

The aim of claim c) is to assess the effects of at least one ORF identified in step b) on the microbiome influenced by roots of said plant,preferably on the rhizomicrobiome of said plant.

The micropeptide provided in step c) may be obtained by chemical synthesis or by recombinant technology.

The micropeptide is preferably provided in the form of a composition comprising said micropeptide.

The micropeptide may be applied on the plant by spraying or watering a composition comprising said micropeptide. Preferably, said composition is applied on the root crown. Applying the composition on the root crown is indeed more efficient than spraying the composition on the whole plant.

The term "root crown" as used herein refers to the part of the plant between the root system and the stem. Since roots and stems have quite different anatomies, major vascular changes take place in this part of the plant.

A plant treated in the same manner and with the same composition, except the presence of the micropeptide, is also used, in order to compare the microbiome influenced by roots of a plant in the presence or absence of said micropeptide.

The micropeptide is preferably applied once the plant has reached 6 leaves, for example after at least two weeks of growth.

The micropeptide may be applied once to seven times a week and/or during one to 6 weeks.

The micropeptide may be applied twice to four times a week, for example three times a week, and/or during two to four weeks, for example during three weeks.

The micropeptide is preferably applied three times a week, during three weeks.

The composition comprising the micropeptide may be sprayed on the leaves and/or on the root crown.

The composition comprising the micropeptide is preferably carefully watered or sprayed on the soil, at the base of the stem, i.e. on the root crown.

The composition may for example comprise at least 1 µM of the micropeptide, preferably at least 5 µM.

The composition comprising said micropeptide preferably comprises an aqueous solution and the micropeptide. The aqueous solution is preferably water.

The composition preferably comprises the micropeptide and water.

500 µL of the solution comprising the micropeptide at a concentration of 20 µM in water are for example used.

When the micropeptide is not soluble in water, an organic solvent may be used to dilute the micropeptide, before adding water. The composition may thus comprise an organic solvent, a micropeptide and water.

In step c), the plant is obviously not the plant of step a), but is a plant of the same genus and species as the plant used in step a).

The plant may be grown in normal conditions and/or in specific conditions.

Normal conditions and specific conditions are as defined above.

The plant is preferably grown in the same conditions as those used in step a), in the case of alternative (i).

In a preferred embodiment, each micropeptide is assessed on a different plant.

Alternatively, a combination of at least two micropeptides may be assessed on the same plant.

### Step d)

Step d) comprises comparing the microbiome influenced by roots of the plant in the presence or absence of said micropeptide. Preferably, step d) comprises comparing the rhizomicrobiome of the plant in the presence or absence of said micropeptide.

Step d) may be carried out at least two weeks after the first application of the micropeptide onto the plant, preferably at least three weeks after the first application of the micropeptide onto the plant, for example three weeks after the first application of the micropeptide onto the plant.

Step d) may comprise determining the genus and/or species of microorganisms and, optionally, their proportion, quantity and/or concentration, in a sample of the roots and/or rhizosphere of the plant in the presence of said micropeptide and in a sample of the roots and/or rhizosphere of the plant in absence of said micropeptide.

Step d) may thus for example comprise measuring an alpha-diversity index, such as Observed Species, Shannon or Simpson index and/or by quantifying bacteria and fungi, for example by qPCR using 16S rRNA for bacteria and/or using ITS2 for fungi.

A change in the alpha-diversity index or in the proportion, quantity and/or concentration of at least one microorganism in the presence versus in the absence of the micropeptide indicates an alteration of the microbiome influenced by roots of the plant.

In particular, a change in the alpha-diversity index or in the proportion, quantity and/or concentration of at least one microorganism of the rhizosphere in the presence versus in the absence of the micropeptide indicates an alteration of the rhizomicrobiome of the plant.

### Step e)

Step e) comprises selecting at least one micropeptide able to alter the microbiome influenced by roots of the plant. Preferably, step e) comprises selecting at least one micropeptide able to alter the rhizomicrobiome of the plant.

The method may further comprise applying the micropeptide selected in step e) onto the plant, in the presence of at least one pathogen and/or at least one pollutant and/or in a stress condition, such as a drought stress, to assess if the alteration of the microbiome influenced by roots of the plant induces a phenotype of interest.

The phenotype of interest may for example be selected from the group consisting of a resistance to at least one plant pathogen, a resistance to at least one stress, a resistance to at least one pollutant and their combinations.

The resistance to at least one stress may for example be resistance to drought stress or resistance to excess of water.

### Method for altering the microbiome influenced by roots of a plant

The present invention also relates to a method for altering the microbiome influenced by roots of a plant, wherein said method comprises administering to said plant at least one micropeptide as defined above. The present invention also relates to a method for altering the rhizomicrobiome of a plant, wherein said method comprises administering to said plant at least one micropeptide as defined above.

As explained above, the micropeptide as defined above induces the transcription of the corresponding miRNA in the plant cells. Said miRNA is then secreted by the plant cells and alters the microbiome influenced by roots of a plant, by acting on at least one microorganism by gene silencing or activation.

An alteration of the microbiome influenced by roots of a plant is particularly as defined above.

The micropeptide may be provided in the form of a composition as defined above or in a container as defined above. Preferably, said micropeptide is applied on the root crown. Administering at least one micropeptide to a plant may comprise spraying or watering said micropeptide on the soil, at the base of the stem of the plant, i.e. on the root crown.

The micropeptide may also be sprayed on the leaves and/or on the root crown.

The micropeptide is preferably applied once the plant has reached 6 leaves, for example after at least two weeks of growth.

The micropeptide may be applied once to seven times a week and/or during one to 6 weeks.

The micropeptide may be applied twice to four times a week.

Altering the microbiome influenced by roots of the plant preferably induces a phenotype of interest in the plant.

The phenotype of interest may selected from the group consisting of a resistance to at least one plant pathogen, a resistance to at least one stress, a resistance to at least one pollutant and their combinations.

The resistance to at least one stress may for example be resistance to drought stress or resistance to excess of water.

For example, plants treated with micropeptide miPEP159c of sequence SEQ ID NO: 3 show a better tolerance to water stress.

### Use of a micropeptide as a plant microbiome modulating agent

The present invention also relates to the use of a micropeptide as defined above as an plant microbiome modulating agent, in particular to induce a phenotype of interest in a plant.

By "plant microbiome modulating agent", it is herein meant a compound able to alter the microbiome influenced by roots of a plant.

The plant microbiome modulating agent is preferably a rhizomicrobiome modulating agent.

By "rhizomicrobiome modulating agent ", it is herein meant a compound able to alter the rhizomicrobiome of a plant.

The phenotype of interest may be as defined above in the section "Method for altering the microbiome influenced by roots of a plant".

Said micropeptide may be provided in the form of a composition as defined above or in a container as defined above.

The invention will be further illustrated by the following figures and examples. However, these examples and figures should not be interpreted in any way as limiting the scope of the present invention.

### Brief description of the sequences

Sequence SEQ ID NO: 1 corresponds to the amino acid sequence of micropeptide 159a (miPEP 159a) of Arabidopsis.
Sequence SEQ ID NO: 2 corresponds to the amino acid sequence of micropeptide159b (miPEP 159b) of Arabidopsis.
Sequence SEQ ID NO: 3 corresponds to the amino acid sequence of micropeptide159c (miPEP 159c) of Arabidopsis.
Sequence SEQ ID NO: 4 corresponds to a scrumbled amino acid sequence of micropeptide miPEP 159c (AtSC159c).
Sequences SEQ ID NO: 6 to SEQ ID NO: 10 correspond to primers used in the Example.

### Figures

Figure 1: Analytical flowchart for the identification of rhizospheric microRNAs
Figure 2: Average number of microRNAs reads for *Arabidopsis thaliana* and *Brachypodium distachyon* rhizospheres and unplanted soils
Figure 3: (A) Venn diagram showing microRNAs sequenced in the associated rhizosphere of *Arabidopsis* and *Brachypodium,* and in unplanted soil. x, y and z are the occurrences of microRNAs annotated as neither of these plants. (B) Core microRNAs identity found in *Arabidopsis* and *Brachypodium* rhizopheres
Figure 4: Phenotype of *Arabidopsis* small RNA mutants. *Ago1-27, dcl1.2, hen1-4, RTL 1* and *RTL1myc* are mutants of *Arabidopsis thaliana* which have an impaired production of small RNAs, including microRNAs. They can be compared to « WT6.5 » which is the wild-type of *A. thaliana.*
Figure 5: Alpha-diversity indices (Observed species, Shannon and Simpson) of bacteria present in (i) root and close rhizosphere samples, (ii) distant rhizosphere of mutants, wild-types and control soils.
Figure 6: Alpha-diversity indices (Observed species, Shannon and Simpson) of fungi present in (i) root and close rhizosphere samples, (ii) distant rhizosphere of mutants, wild-types and control soils.
Figure 7: Bray-Curtis distances-based ordination of bacterial communities in roots and rhizosphere of mutants, WT and Control soil.
Figure 8: Weighted UniFrac distances-based ordination of bacterial communities in roots and rhizosphere of mutants, WT and Control soil.
Figure 9: Bray-Curtis distances-based ordination of fungal communities in roots and rhizosphere of mutants, WT and Control soil.
Figure 10: Weighted UniFrac distances-based ordination of fungal communities in roots and rhizosphere of mutants, WT and Control soil.
Figure 11: Hairpin structure of the pri-miRNA 159c. This pri-miRNA encodes miPEP159c located at the 5' end, and the mature miRNA159c and miRNA159c*.
Figure 12: Comparison of phenotypic changes of miPEP159a, miPEP159b and miPEP159c-treated plants after 3 weeks of treatment. The fresh weight of the leaves was statistically tested with a Wilcoxon test, compared to the control (p<0.001 = ***, ns= non-significant).
Figure 13: Alpha-diversity indices (Observed species, Shannon and Simpson) of bacteria and archaea present in (i) root and close rhizosphere samples, (ii) distant rhizosphere and (iii) unplanted soils, treated with miPEP-A, miPEP-B, miPEP-C or water (Control-mi-PEP).
Figure 14: Alpha-diversity indices (Observed species, Shannon and Simpson) of fungi present in (i) root and close rhizosphere samples, (ii) distant rhizosphere and (iii) unplanted soils, treated with miPEP-A, miPEP-B, miPEP-C or water (Control-mi-PEP).
Figure 15: Principal Coordinate Analysis of Bray-Curtis distances of bacterial communities in i) root and close rhizosphere, ii) distant rhizosphere and iii) unplanted soil samples.
Figure 16: Principal Coordinate Analysis of Bray-Curtis distances of fungal communities in i) root and close rhizosphere, ii) distant rhizosphere and iii) unplanted soil samples.
Figure 17: Principal Coordinate Analysis of Weighted UniFrac distances of bacterial communities in i) root and close rhizosphere, ii) distant rhizosphere and iii) unplanted soil samples.
Figure 18: Principal Coordinate Analysis of Weighted UniFrac distances of fungal communities in i) root and close rhizosphere, ii) distant rhizosphere and iii) unplanted soil samples.
Figure 19: Transmission electron microscopy (TEM) of *A. thaliana* roots grown in petri dishes and a chemically fixed sample of rhizodermis cells (A and B) showing high vesicle synthesis activity. Roots grown in soil: rhizospheric bacteria (white arrows) seem to cluster near areas of vesicle accumulation (black arrows) in the roots (C and D). Detail of a typical zone of rhizospheric bacteria (from roots grown in soil) attached to the cells of the rhizodermis (E). An enlargement of the frame (E), shows the colocalization of a rhizospheric bacterium and an extracellular vesicle (F).
Figure 20: Microscopy of cryo-fixed root samples showing a high concentration of bacteria in contact with rhizodermis cells (A). Enlargement of the frame (A) shows bacteria grouped together embedded in a biofilm (dark grey arrow), nearby a high accumulation of vesicles (black arrows) (B).
Figure 21: Workflow of differential centrifugations and density gradient for exosome isolation from the rhizosphere, followed by RNA extraction
Figure 22: Average size of exosomes measured by Nanoparticle Tracking Analysis (NTA) (A).
Figure 23: RNA extracted from samples taken at each exosomal purification step: the first one from the filtrate after filtration on gauze (P1), the second after concentration on an Amicon^{®} column (P2) and the third on the pellet obtained at 120,000 g (P3).

### Example: Implication of microRNAs in plant-microbe interactions in the rhizosphere & the role of miRNA159c in water stress tolerance with applied miPEP159c in Arabidopsis thaliana

Currently, rhizosphere engineering is carried out either (i) by addition of molecules of primary or secondary metabolism to promote the development of the microbiome, or (ii) by inoculation of bacterial or fungal strains selected for their beneficial impact on the plant. The first technique is non-specific and allows the development of both beneficial and potentially negative microorganisms. The second technique has two major problems, either the strain is invasive and destabilizes the beneficial balance of the whole microbiome, or it is unable to maintain itself in a highly competitive ecological system.

The invention, based on the manipulation of microRNAs, can target in a very specific way one or several strains to activate or inhibit their development, without going the need of genetic modification. This method is an interesting ecological solution for an environmental application because it will allow a very targeted manipulation of the microbiome associated with the plant, by inhibiting strains with a negative effect and/or by favoring strains with a beneficial effect.

### Materials and methods

### Plant material

*Arabidopsis thaliana* Columbia plants (Col-0) were used as the dicotyledonous model, whereas *Brachypodium dystachion 21-3* plants (Wild Type) were used as the monocotyledonous model. Plants were grown in a greenhouse maintained at 20°C in a mixture of ¾ horticultural soil and ¼ sand, sifted to 2.2 mm. The plants were exposed to 16 hours of light per day and 8 hours of darkness. Both plant species were grown for 4 weeks before their rhizospheres were harvested and RNA extracted. Unplanted soils, prepared similarly as to those used for *Arabidopsis* and *Brachypodium,* were placed in the same conditions and used as control soil. Mutants *ago1-27, dcl1-2, hen1-4, RTL1* and *RTL1myc* were also grown in the same conditions as described before.

### miPEP design and synthesis

microRNA-encoded peptides (miPEPs) were designed with the following amino acid sequences: MTWPLLSLSFLLSKYV (miPEP 159a, SEQ ID NO: 1), MGLRKVLEMNTIFDSLFLSH (miPEP 159b, SEQ ID NO: 2) and MQNLRVHVFLIESARC (miPEP 159c, SEQ ID NO: 3). To validate the specific action of miPEP159c, a scrambled miPEP, with a random amino acid sequence, was used as a supplementary control: IHSQEMLRVCRALFNV (AtSC159c, SEQ ID NO: 4). These peptide sequences are encoded in pri-miRNA from *Arabidopsis thaliana* and were identified using PACBio iso-seq sequencing. The first ORF-encoded peptide was chosen to synthesize miPEPs.

### miPEP treatment

In order to evaluate the role of the abundant miR159 found in the rhizosphere, *Arabidopsis thaliana* and unplanted soils were treated with miPEP159a, miPEP159b, miPEP159c or water (control group), resulting in 16 biological samples per treatment. The treatment consisted of watering the plants and soils with 500µL of a miPEP solution (20µM), three times a week, during three weeks. Plants were carefully watered on the soil, at the base of the stem. On the first treatment day, the plants were at a 6-leaves stage.

### Rhizospheric soil sampling, miRNAs extraction and sequencing

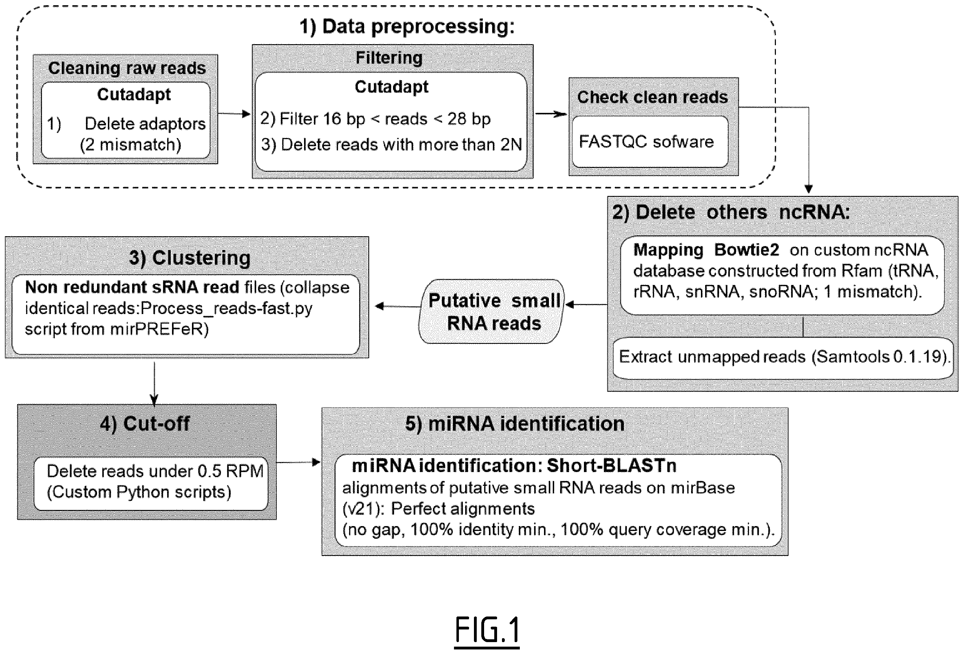

Roots of one-month old plants were harvested, the excess of soil was discarded, and the soil located at the vicinity of roots was recovered and considered as the rhizospheric soil. Roots were then washed in PBS solution. The soil samples from the unplanted pots were also harvested. These samples were flash-frozen in liquid nitrogen and stored at -80°C until RNA extraction. RNA extraction was performed with TRIzol^{®} reagent (Sigma-Aldrich). Small RNA-Seq and data preprocessing were performed as previously described in Cave-Radet et al. (2019 « Evolution of Small RNA Expression Following Hybridization and Allopolyploidization: Insights from Spartina Species (Poaceae, Chloridoideae) », Plant Molecular Biology 102, n° 1-2 (janvier 2020): 55-72). Sequencing was performed single end, on a NextSeq500 (Illumina^{®}) sequencer (1x50 pb, 20 million reads). RNA-Seq was performed for all 9 samples. Libraries were prepared using 1 µg of total RNA, with the « NEBNext^{®} Ultra^{™} II DNA Library Prep Kit » kit (Illumina^{®}). Bioinformatic analyses were conducted on the GenOuest Bioinformatic Platform (Biogeneouest, University of Rennes1) (see analytical flowchart in **Figure 1****).** Small-RNA raw reads were first trimmed by Cutadapt to remove 3' sequencing adapters (2 mismatches allowed). Only reads between 16 to 28 nucleotides with less than 2 unknown bases were retained as clean reads. Remaining clean reads were mapped by Bowtie2 on a custom database containing Rfam non-coding RNA families' sequences (nc-RNA: tRNA, rRNA, snRNA and snoRNA). Mapped reads (allowing for 0 or 1 mismatch) were discarded (Samtools 0.1.19) to remove other nc-RNAs and construct putative small-RNA datasets. Finally, identical reads were collapsed among libraries to get non-redundant putative small-RNA datasets (process_reads-fasta.py script from mirPREFeR). For downstream analyses, only reads representing at least 0.5 RPM (Reads Per Million; *e.g.* 10 reads minimum from 20 million reads sequencing depth) were kept to remove low coverage small RNA reads. Non-redundant putative small-RNA reads were then aligned by BLAST+, using short-BLASTn on mirBase (version 21). Alignments were filtered to retain the best blast hit with perfect match (full length, no gap, and 100% identity and query coverage) and identify miRNAs candidates.

### Rhizospheric Extracellular Vesicles purification

### A.thaliana and B.distachyon plants were grown in a mixture of ¾ horticultural soil ¼ sand sifted to 2.2 mm in seedling plates containing 160 cells (cell: 30mm in diameter and 50mm deep)

The roots with their rhizosphere (20 g with about 1mm of soil still on the roots) are harvested and are placed in PPCO Nalgene ^{™} centrifuge flasks (ThermoScientific ^{™}) with 100ml of autoclaved 1X PBS and the whole stirred for 1 hour, in the presence of sterile glass beads. The extract was then filtered through a gauze before being centrifuged at 2000g at 4°C for 20 min, the supernatant is then transferred to a new flask to be centrifuged at 4000g at 4°C for 30 min. The supernatant is once again transferred to Oak Ridge Tubes PolyCarbonate 35 mL Conical Nalgene ^{™} (Thermo Scientific ^{™}) then centrifuged at 10,000g for 1 hour at 4°C. The supernatant (approximately 85mL) was concentrated on Amicon^{®} Ultra-15 Centrifugal Column Filters Ultracel^{®} 100kD, with successive centrifugations of 1000g for 15 minutes until a volume of about 8.9mL was obtained. The extract thus concentrated is transferred to an Optiseal^{®} Polypropylene Centrifuge tube (16x60 mm) and was ultracentrifuged on a 90 Ti Fixed-Angle Titanium Rotor for 1 hour at 120,000g at 4°C. The supernatant is discarded and the pellet was diluted in 500µL of 1X PBS. 100 µL were used to check the quality of the vesicles by NTA, and the remainder sample was used for RNA extraction.

Furthermore, to monitor the presence of vesicles containing miRNAs throughout the protocol, three samples were taken from the extract at different stages: the first from the filtrate after filtration on gauze (P1), the second after concentration on a column Amicon^{®} (P2) and the third on the pellet obtained at 120,000g (P3).

### Nanoparticle Tracking Analysis (NTA)

NTA measurements were carried out with Nanosight LM10 device system equipped with a 40 mW laser operating at λ = 638 nm. Video footage was recorded via a CCD camera at 30 images per second and analyzed via the NANOSIGHT NTA 2.0 Analytical software 'Software Suite'. A measurement blank with pure water was carried out beforehand. Each sample was subjected to three acquisitions of 60 seconds at 25°C. Hydrodynamic calculations were based on the viscosity of water.

### Nanodrop essays of EVs nucleic acids

8 µl of the purified vesicles were treated with 2 µl of 1X Triton. The samples were run through the Nanodrop at λ = 260nm.

### DNA extraction and metabarcoding

The mutant plants were sampled in the following manner, resulting in two types of samples: the roots were quickly rinsed with sterile PBS 1X, the roots and closely attached rhizosphere were dried with paper towel and directly frozen in liquid nitrogen, whereas the leftover, more distant, rhizospheric soil was frozen with the rinse solution. During the miPEP experiment, plants were sampled in a similar manner, however each sample corresponds to the pooling of two plants/soils. Unplanted soils were also analyzed after treatment. In order to extract DNA, plant samples were first ground in liquid nitrogen with a sterile mortar and pestle, then processed with the Nucleospin Plant II kit. DNA from soil samples was extracted. First, to each sample, 500µL of a lysis buffer were added. This buffer being composed of 50% potassium solution (86% K2HPO4, 14% KH2PO4, pH 8.0) and 50% of a solution of 10% Cetyltrimethylammonium bromide (CTAB) and - 0,7 M NaCl. An additional 500µL of phenolchloroform-isoamyl alcohol (25:24:1, pH 8.0) was added and then samples were ground by bead beating at 30 m.s-1 for 3 min, using Lysing Matrix E tubes (MP Biomedicals, California, USA). The lysed samples were then centrifuged at 14000g for 10min at 4°C, the aqueous phase was retrieved and 500µL chloroform-isoamyl alcohol (24:1) were added, before repeating centrifugation. To the aqueous phase, 1mL of 30% PEG 6000 - 1.6 M NaCl was added and left overnight at 4°C to precipitate. The samples were then centrifuged again for 30min in order to pellet the nucleic acids. The pellet was then washed with cold 70% ethanol and centrifuged for 10min. The supernatant was discarded and the pellet left to dry for 10min, before adding 50µL of molecular biology graded water. DNA quantity and quality were analysed using Nanodrop spectrophotometer (ND-1000) and by running 1% agarose gels. For each sample, 10µL of DNA were sent off for PCR amplification, library construction and MiSeq Illumina sequencing (2*300 bp). Bacteria and archaea were sequenced by amplifying the V4-V5 region 16S rRNA gene with the following primers F-(5'-GTGYCAGCMGCCGCGGTAA-3') (SEQ ID NO: 5) and R-(5'-CCGYCAATTYMTTTRAGTTT-3') (SEQ ID NO: 6) and fungi with primers ITS9-F-(5'-GAACGCAGCRAAIIGYGA-3') (SEQ ID NO: 7) and ITS4-R-(3'-TCCTCCGCTTATTGATATGC-3') (SEQ ID NO: 8).

### Processing sequences and generating ASVs

After sequencing 16S and ITS ribosomal RNA gene amplicons, the corresponding sequences were processed, by the sequencing center, using the bioinformatic pipeline AmpliconTagger: DNA reads have to pass a quality control and be filtered. After these initial processing steps, reads which are 100% similar are clustered together and are assigned taxonomy by using the RDP classifier and the Greengenes database. The abundance of each cluster and their assigned taxonomy result in an ASV (Amplicon Sequence Variants) table, which is used in the following microbial analyses. The initial filtrations resulted in 3,547,108 sequences, which passed the quality control and 545 classified clusters, for fungi, and 3,776,292 sequences and 4,023 classified clusters, for bacteria and archaea.

### Microbial diversity and structure analyses

Before starting the microbial analyses, an additional filtration step was added: ASVs that were present in less than 2 samples were removed. To evaluate the diversity within our samples, alpha-diversity indexes were measured (e.g. Observed Species, Shannon, Simpson) on rarefied data. Fungal sequences were normalized at 814 sequences, eliminating 3 samples, and bacterial/archaeal sequences were normalized at 6538 sequences. After checking for data normality, a linear model was used to test for statistical differences. If data was not normal, a general linear model from Gamma family, with a "log" link was the best suited test to perform. To compare the similarity of microbial communities between samples, beta-diversity was measured, on non-rarefied data, through 3 types of analysis: Bray-Curtis (based on ASV abundance, no phylogeny); Weighted Unifrac (based on ASV abundance and phylogeny) and Unweighted Unifrac (based on presence/absence of ASV and phylogeny). These measurements were represented as PCoAs (Principal Coordinate Analysis) and were statistically tested through the Adonis function (Permutational Multivariate Analysis of Variance Using Distance Matrices). A permutation test was then performed to evaluate the multivariate homogeneity of group dispersions, with the betadisper function. If data did not pass this test, an Anosim test was preferred to an Adonis test, as it does not assume equal group variance. To determine differential ASV abundances between conditions, the DeSeq2 package was used, showing results with p.value<0,01. Indicator species were determined with the indicspecies package and the multipatt function.

### Microbial quantification by qPCR

In each sample, bacteria/archaea (16S rRNA) and fungi (ITS2) were quantified in order to create a more comprehensive microbial analysis. These genes were quantified by qPCR, in triplicates, on the Roche LightCycler 480 system. Each reaction was composed of 1,8µL of molecular biology graded water, 0,1 µL of each primer (10µM) and 4µL of SYBR Green I Master mix, in addition to the 2µL of DNA (diluted to 25ng/µL), resulting in a final volume of 8µL per well. In soil sample mixes, 0,67mg/mL of BSA (i.e. 0,268µL/well) were added to help with amplification. The primers used for fungal quantification were the same as those used for sequencing. Those used for bacteria and archaea are the following: 341F (5'-CCTACGGGNGGCWGCAG- 3') (SEQ ID NO: 9) and 534R (5'-ATTACCGCGGCTGCTGGCA - 3') (SEQ ID NO: 10). The qPCR program for fungi started with a pre-incubation phase (95°C- 4min), followed by amplification (95°C - 30s ; 49°C - 1min ; 72°C - 1min), repeated over 40 cycles, finished with an extension phase (72°C - 10min) and a melting curve from 49°C to 97°C, with an increase of 0.5°C/5s. The program for bacteria started with a pre-incubation phase (95°C- 5min), followed by amplification (95°C - 15s ; 60°C - 30s ; 72°C - 30s; 80°C - 5s), repeated over 34 cycles, finished with a melting curve from 65°C to 95°C, with an increase of 0.5°C/5s. Quantification was performed with a standard curve, created by serial dilutions of 16S/ITS inserted plasmids with a known number of copies/µL. These series ranged from 10⁸ to 10² copies for ITS plasmids (R²=0,9987 et R²=0,9882 avec BSA) and 10⁶ to 10² copies for 16S plasmids (R²=0,994 et R²=0,9968 avec BSA). Once Ct values were converted to copies/µL, dilution factors were considered and the number of fungal copies was corrected with a ratio equal to the fungal ASV/non-fungal ASV, for each sample, found during sequencing, as ITS9-4 primers were discovered to not be specific to fungi. In the ASV table, abundances were converted in proportion to the quantity of 16S/ITS found in each sample *(i.e.* in 50ng of DNA).

### Chemical fixation and preparation of biological sample for Transmission Electron Microscopy (TEM)

Fresh *A.thaliana* roots of about 1 cm, from soil or agar grown plants, were prepared. The samples were dipped in PAF fixative solution (Paraformaldehyde 2%, glutaraldehyde 2%, phosphate buffer 0.05M pH 6.8) for 24 hours in a desiccator connected to a vacuum pump. The vacuum is applied for 2 minutes then slowly the air is reintroduced. The operation is repeated until the samples fall to the bottom of the container. After 24 hours, a quick wash followed by two 5-minute washes with phosphate buffer were performed, which will then be stirred for 1 hour in a 0.02g / ml solution of tannic acid (diluted in phosphate buffer). A quick wash followed by two washes of 5 minutes in phosphate buffer. For post fixation, the samples were immersed in 2% osmium (1 vial of osmium of 4% (2mL) + 2mL of phosphate buffer) then stirred for 1 hour. This step was followed by quick wash and by two 10-minute washes in phosphate buffer before being transferred to a new pill container. The first washing step is done with EtOH 50% which includes a quick wash and a second of 15 minutes, then a quick wash and a 15-minutes wash with 70% EtOH. From this concentration a background of liquid should always remain in the pill organizer. The next step is performed with 80% EtOH with a quick wash and two 15-minutes washes. The same step is carried out with 90% EtOH with one quick wash and two 15 minutes washes. Then a quick wash and three 15-minutes washes are performed with 100% EtOH. The samples were then impregnated in the resin in three baths: the first bath composed of 50% HPMA and 50% Resin for 1 hour 30 minutes, the second bath of 1/3 HPMA and 2/3 of resin for 1h30 then in the resin alone for a full night. Additional Impregnation was carried out the next day in the resin alone for 2 to 3 hours. Then the samples were included in a new resin, which was cured for 36 h at 60 ° C. The resin sections were made with a microtome or with a glass knife and the observations were carried out with a GEOL - JEM 1400 electron microscope at the MRic platform (Microscopy Rennes Imaging Center).

### Cryo-fixation by High Pressure Freezing (HPF) and preparation of samples for MET observation

Root section samples were cryo-fixed by EMPACT2 HPF (Leica Microsystems), fixed at - 90°C with 0.25% uranyl, 2% osmium and 0.25% glutaraldehyde in acetone. The samples were then impregnated in EPON resin baths of successive increasing concentrations (25%, 50%, 75% and 100%) at -30°C, 2 hours each time. Once the samples have returned to room temperature, they are polymerized for 24 hours at 60°C.

### Results

### Roots exporting microRNAs in the rhizosphere is a common feature in plants

Our experiment aimed at determining if roots export miRNAs in the rhizosphere, and if this is a common feature in plants. To attain this objective, we focused our work on two evolutionary divergent plant models, *Arabidopsis thaliana* and *Brachypodium distachyon* that were grown in triplicates for one month alongside unplanted controls.

A total of 269,256,210 reads were obtained for all samples (average of 33,657,026 reads per sample), from which 182,121,045 reads (average of 22,765,131 reads per sample) were retained after preprocessing (see **Table 1** for average values per sample after each key step).

**Table 1: Average number of sequencing reads after key analytical steps**

| | Arabidopsis | | Brachypodium | | Soil | |
|---|---|---|---|---|---|---|
| | Reads | %raw | Reads | %raw | Reads | %raw |
| Raw reads | 33,474,870 | 100% | 32,270,061 | 100% | 34,763,826 | 100% |
| Clean reads | 21,605,678 | 65% | 20,679,776 | 64% | 25,314,919 | 73% |
| Putative sRNA reads | 6,479,801 | 19% | 6,131,658 | 19% | 8,329,488 | 24% |
| Unique putative sRNA reads | 4,101,872 | 12% | 4,028,960 | 12% | 5,029,405 | 14% |
| Unique putative sRNA reads (0.5 RPM cut-off) | 12,907 | 0.04% | 15,067 | 0.05% | 26,793 | 0.08% |

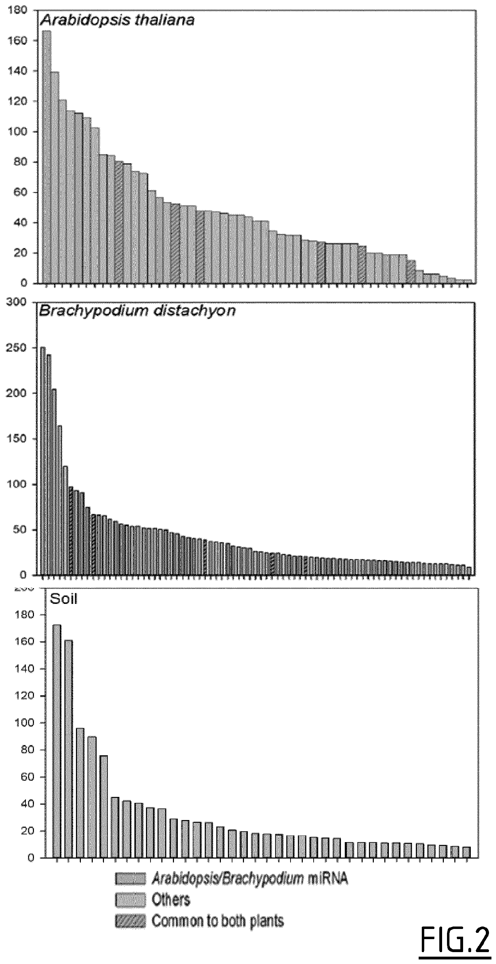

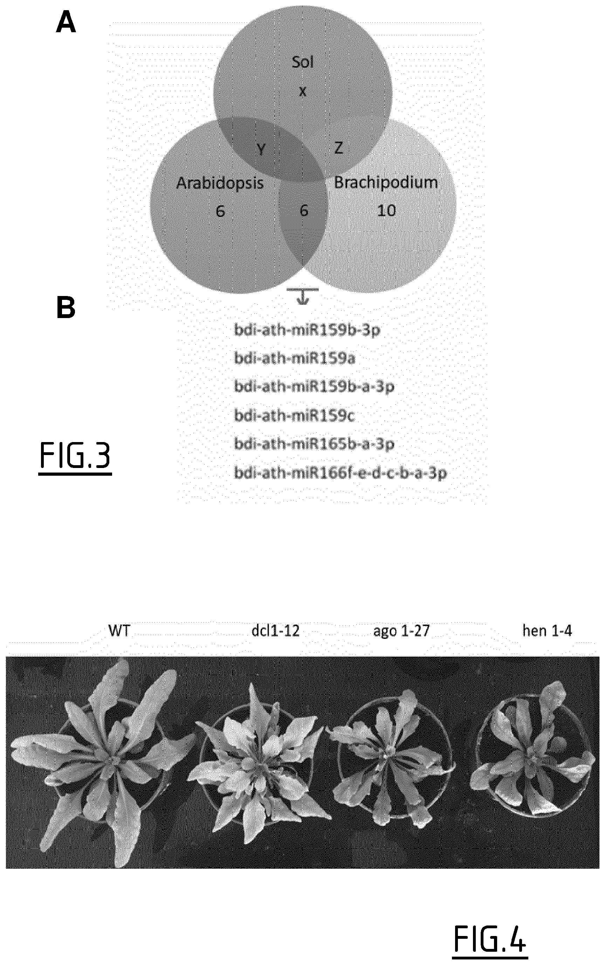

Between 3,450,952 and 9,500,056 unique putative small RNA reads per library were kept after filtering for other nc-RNA filtering and redundancy removal. Only between 12,455 and 29,729 reads per sample passed the 0.5 RPM threshold. From the BLAST alignments on mirBase, we identified 54 putative miRNAs from *A. thaliana* rhizosphere extracts (12 annotated as *Arabidospsis),* 79 putative miRNAs from *B. distachyon* rhizosphere extracts (16 annotated as *Brachypodium),* and 37 putative miRNAs in the unplanted soil (no miRNAs annotated as *Arabidopsis* or *Brachypodium)* **(****Figure** 2). Among the 12 miRNAs annotated as *Arabidopsis* in the *Arabidopsis* rhizosphere and the 16 miRNAs annotated as *Brachypodium* in the *Brachypodium* rhizosphere, 6 were shared by both plant species **(****Figure** 3). These common miRNAs were annotated as bdi-ath-miR159b-3p, bdi-ath-miR159a, bdi-ath-miR159b-a-3p, bdi-ath-miR159c, bdi-ath-miR165b-a-3p, bdi-ath-miR166f-e-d-c-b-a-3p. The potential targets within soil bacterial genomes for these 6 plant miRNAs are currently being searched using the psRNATarget algorithm and the RefSoil database.

This preliminary data confirms that miRNAs are present in large quantities and stable in the soil environment, even in the absence of plants. The miRNAs were apparently not the result from the degradation of RNA as they passed all the very stringent quality filtering steps of our pipeline and had perfect matches with known miRNAs from the mirBase database. The stability of the miRNAs in the mammalian gut was previously linked to the binding by proteins and their packaging in extracellular vesicles for transport. It remains to be seen if the same protection mechanisms could be at play in the rhizosphere and in the soil.

Furthermore, our preliminary experiment allowed us to identify specific miRNAs that were found in the rhizosphere of *Arabidopsis* and *Brachypodium.* Our data strongly supports the plant origin of some of the miRNAs identified. Firstly, no microRNA matching with *Arabidopsis* or *Brachypodium* genomes were found in the soil without plants suggesting that plants deeply impact the composition of soil microRNAs. Additionally, a previous study showed that among the 243 known *Arabidopsis* miRNAs, 133 were found in roots tissues and the 12 miRNAs annotated as *Arabidopsis* in the *Arabidopsis* rhizosphere were a subset of these 133 root miRNAs. Most interestingly, we found that *Brachypodium* and *Arabidopsis* rhizosphere harbored 6 miRNAs in common, suggesting that distantly related plants (eudicot vs. monocot) potentially share a common core miRNA set used to interact with the rhizosphere microbiota. Inversely, the miRNAs that were not shared between the two plants might represent species-specific interaction pathways and could partly explain the differences observed in the microbiota composition and activities between different plant species. Interestingly, some of the miRNAs that were shared between *Arabidopsis* and *Brachypodium* were previously reported having important roles in the roots or in plant-pathogen interactions. For instance, the accumulation of miR165 was previously shown to result in the elongation of *Arabidopsis* roots, which could indirectly affect the rhizosphere microbiota. A previous study showed that miR166 and miR159 were produced by cotton plants in response to fungal infection and were then exported to the fungal hyphae to silence specific genes resulting in a reduced virulence of the fungus.

The preliminary data presented here clearly highlights that specific miRNAs are stably excreted in the rhizosphere of plants. Some of these miRNAs appear to be shared among various plant species. The exact role these miRNAs might have is very intriguing and completely unknown. We further hypothesize that the plant miRNAs secreted in the rhizosphere are taken up by microorganisms, selectively modifying microbial gene expression, leading to shifts in the microbiome composition, functions and activities.

### Mutations in plant small RNA pathways alter rhizospheric microbiota

To examine a previous hypothesis that plants excrete in their rhizosphere small RNAs, such as miRNAs, and by doing so, regulate their rhizospheric microbial communities, 5 different *Arabidopsis thaliana* mutants were grown **(****Figure 4****)** and their rhizospheres were sequenced to identify the microbes present. The mutants used in this experiment were the following: (i) *ago1-27* mutant which has partially impaired AGO1 function, rendering post-transcriptional gene silencing completely deficient (affects both siRNA and miRNA pathways); (ii) *dcl1-2* is a DCL1 loss-of-function mutant, which means that the miRNA pathway is disturbed causing low miRNA levels and developmental problems; (iii) *hen1-4* mutant is affected in both siRNA and miRNA accumulation; (iv) *RTL1* mutant over-expresses RTL1 RNAse III preventing siRNA production, but not miRNA; (v) *RTL1myc* mutant over-expresses RTL1, but the protein is flagged with Myc, which renders RTL1 less active, thus the decrease in siRNA levels is less serious than with *RTL 1* mutant. This experiment's purpose is to explore the effects of diverse siRNA and miRNA disruptions on the rhizospheric communities of *A. thaliana.* Evidently, such mutations have multiple effects on the plant's organism, such as developmental issues or other phenotypic modifications (e.g. curled leaves, discoloration...). The effects of these mutations on the microbiota have to be carefully interpreted as they could be indirect effects, due to the pleiotropic nature of these mutations. However, these results can still give preliminary insights regarding the importance of small RNAs in regulating rhizospheric microbial communities, directly or indirectly.

As explained it the material and methods chapter, samples are grouped into 2 types: "ROOTS" refers to the roots and closely attached rhizosphere, these samples include microbes from inside the roots (endophytes), on the rhizoplane and in the close rhizosphere. "SOIL" refers to the more distant rhizosphere, which results from the rinsing of roots, forming a "soil solution". Note that "WT" refers to *A. thaliana* Col-0 wild type plants, whereas "Control" refers to unplanted soils. The following results originate from the combination of 16S and ITS sequencing and quantification by qPCR.

### 1) General composition at the phylum and genus level

### Bacteria/Archaea

In the distant rhizosphere, the 10 most abundant bacterial phyla were, in descending order, Proteobacteria, Bacteroidetes, Planctomycetes, Verrucomicrobia, Acidobacteria, Actinobacteria, Chloroflexi, Gemmatimonadetes and Armatimonadetes. Within the wide diversity of bacterial genera in those samples, the 10 most detected were, in descending order, Devosia (class: Alphaproteobacteria) and Opitutus (class: Opitutae), Rhizomicrobium (class: Alphaproteobacteria), Pir4 lineage (phylum: Planctomycetes), Chitinophaga (class: Chytinophagia) and Subgroup 6CL (phylum: Acidobacteria), CPla-3 termite groupOR (phylum: Planctomycetes) and uncultured Chitinophaga (class: Chytinophagia), and finally Dongia (class: Alphaproteobacteria). This list is far from exhaustive as there are many other annotated genera but also unknown ones within these samples. These bacterial taxa are commonly found in soils all over the world, however Firmicutes phylum is not in the top 10 most abundant phyla in our samples, probably because of the nature of the soil: it originates from the rhizosphere and not unplanted soil.

In the roots and close rhizosphere, the most abundant bacterial phyla are very similar to those found in the distant rhizosphere but in different proportions: Proteobacteria, Bacteroidetes, Acidobacteria, Verrucomicrobia/Actinobacteria/Chloroflexi, then Planctomycetes, Gemmatimonadetes, Spirochaetae and finally Chlorobi. The most abundant genera were Chitinophaga/Rhizomicrobium/Opitutus, Devosia, Rhodanobacter (class: Gammaproteobacteria)/Dongia, uncultured-Chitinophaga/Pseudolabrys (class: Alphaproteobacteria)/Subgroup 6CL. Proteobacteria, Bacteroidetes and Actinobacteria are usually the top 3 phyla found in A. thaliana roots , but our root samples were still associated with their close rhizosphere when sampled, explaining the high amount of Acidobacteria compared with Actinobacteria.

### Fungi

The 4 major fungal phyla in our samples were Ascomycota, Basidiomycota, Chytridiomycota and Zygomycota. Many sequences were classified under an unknown phylum. It is surprising to not find any ASVs related to the Glomeromycota phylum, especially in root samples, as these fungi are essential to plants and found in over 80% of plant roots world-wide. Perhaps, if the sequences were confronted to an AMF (arbuscular mycorrhizal fungi) database, like MaarjAM, more ASVs would be identified as such. In both sample types, the Basidiomycota phylum was the most present: more than 50% of fungal ITS copies in *dcl1-2, hen1-4* mutants, WT and Control. Mutants *ago1-27* and *RTL1myc* had a majority of copies from "unknown" phylum. In *RTL1* mutants, 70% of copies in root and close rhizosphere samples and 42% in the distant rhizosphere were of the Chytridiomycota phylum. In comparison, in roots and close rhizosphere, WT, *hen1-4* and *RTL1myc* plants have only 0,4-2,8% of sequences corresponding to Chytridiomycota phylum, whereas mutants *ago1-27* and *dcl1-2* have around 28%. This phylum seems specifically enriched in *RTL1* mutants. This specificity reflects in the top 10 genera found in our samples: the genus Olpidium (phylum: Chytridiomycota), which is moderately abundant in other samples, is significantly enriched in *RTL 1* in distant rhizosphere and even more so in roots and close rhizosphere. This fungal genus comprises many intercellular parasitic species, which can be pathogenic to plants, animals, fungi and oomycetes. While plants infected by Olpidium species do not show much damage, some species are well known for being phytovirus vectors, which can cause more harm. These fungi are known for completely overtaking fungal communities over the long run: in one study, there was a dramatic increase of Olpidium fungi in the rhizosphere from the 1st to the 3rd generation of *Brassica rapa.* Either these mutants have selected a non-pathogenic species of Olpidium or the *RTL1* mutant seeds were infected prior to the experiment. Otherwise, the most abundant genus is Cristinia (phylum: Basidiomycota, class: Agaricomycetes), and additionally Clitopilus (phylum: Basidiomycota, class: Agaricomycetes) in root samples, both common saprobic fungi.

### 2) Assessing diversity within samples (alpha-diversity)

### Bacteria/Archaea

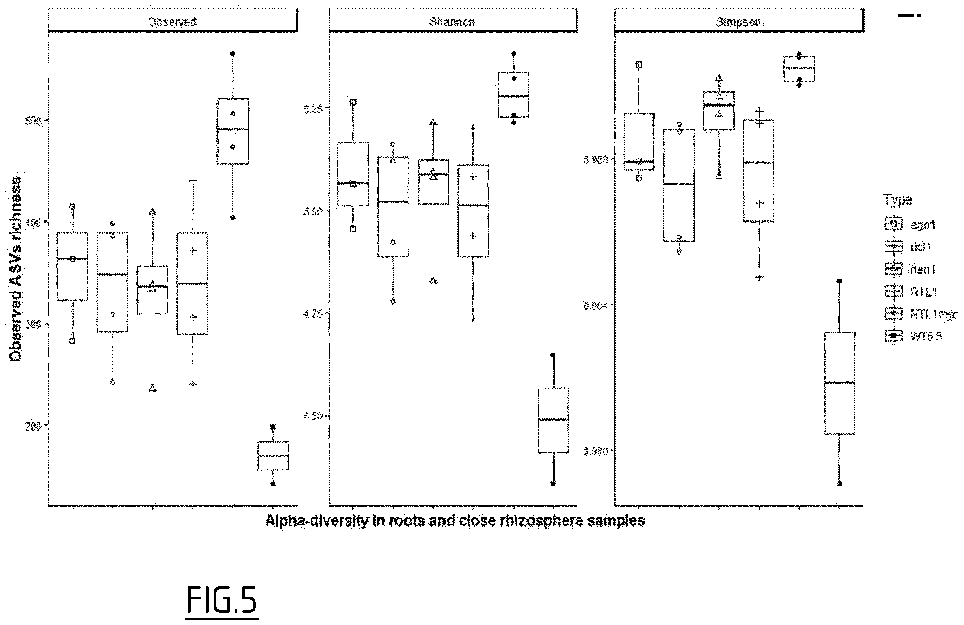

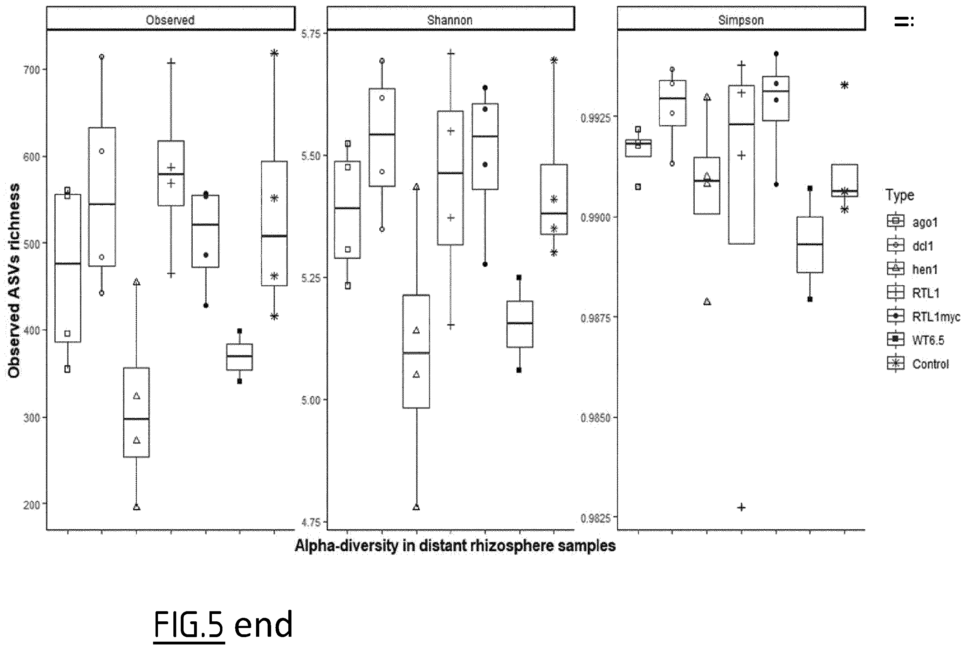

Prior to estimating the alpha-diversity in our samples, the reads were rarefied as described in the previous chapter. In the distant rhizosphere, *RTL1* and *dcl1-2* have a significantly higher number of bacterial observed species (p<0.05), compared to WT, but their Shannon and Simpson indices are less different **(****Figure** 5). This could suggest that these samples have more different species, but less even communities: for example, they could be dominated by only a few. Interestingly, in root and close rhizosphere samples, all mutants have a very significantly higher number of observed species (p<0.05) and diversity indices (p<0.01), compared with WT. *RTL1myc* has the most diverse and even communities.

### Fungi

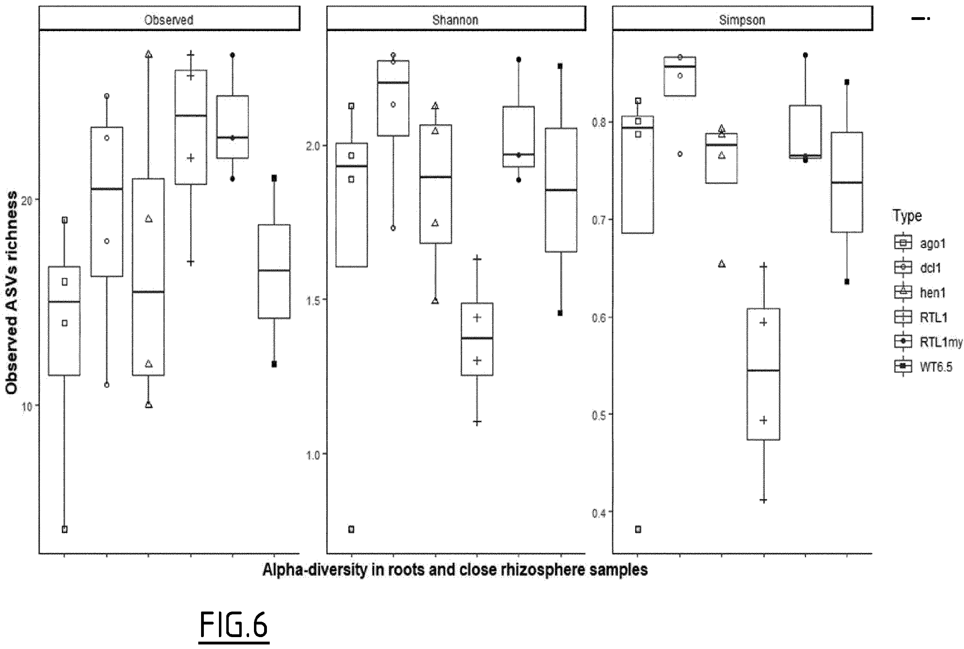

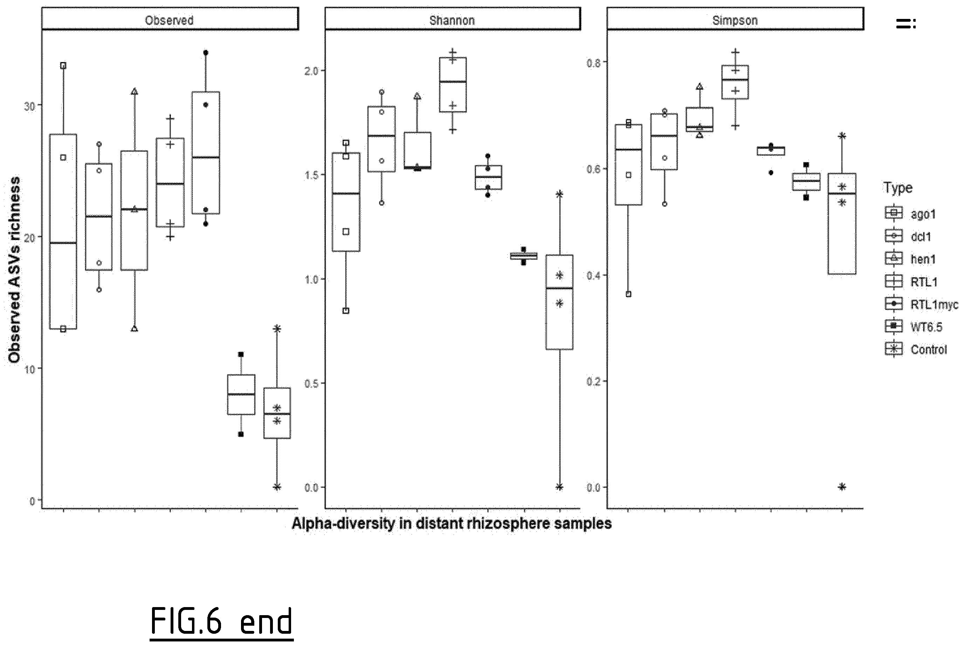

Interestingly, in distant rhizosphere samples, WT and Control (unplanted soil) have similar fungal richness values, compared to the mutants, which all have a significantly higher number of observed species (p<0.05) **(****Figure 6**). When investigating diversity and evenness with the Shannon and Simpson indices, only *RTL 1* mutant is significantly richer than WT (p<0.01 for Shannon). This tendency vanishes in root and close rhizosphere samples: there is no difference in observed species and regarding the other indices, *RTL1* mutant is even slightly less rich than WT (p<0.1 for Simpson). This could be explained as this index considers both diversity and evenness and *RTL 1* mutants seem dominated by a certain species of Olpidium, in root samples.

In conclusion, in roots and close rhizosphere, bacterial communities are significantly more diverse and even than WT, especially *RTL1myc,* whereas fungal communities do not show any differences in diversity. In the distant rhizosphere, bacterial communities are similar in diversity to WT, except for *RTL 1* and *dcl1-2,* which have a higher number of species, but non-even. The fungal communities in the distant rhizosphere are, however, significantly more diverse than WT, especially *RTL1.* The general tendency seems to show that diversity in bacterial communities is more affected by the siRNA and miRNA mutations in roots and close rhizosphere, whereas fungal communities are more affected in the distant rhizosphere. Even though root and soil samples should not be directly compared, due to different DNA extraction methods, it is interesting to note that there were more 16S copies quantified in soil samples VS root samples, and on the contrary, more ITS copies were quantified in root samples VS soil samples. In other words, compartments with less 16S or ITS copies display higher diversity in mutants compared with WT, this could be due to non-sufficient sampling, however, our rarefaction curves show that all samples were sampled enough.

Also, the *RTL 1* mutant stands out as it seems to have the most differences in diversity indices compared to WT.

### 3) Estimating the differences between mutants (beta-diversity)

To investigate more deeply the composition and similarities between communities, beta-diversity was estimated throughout our samples, using both Bray-Curtis and Weighted UniFrac distances.

### Bacteria/Archaea

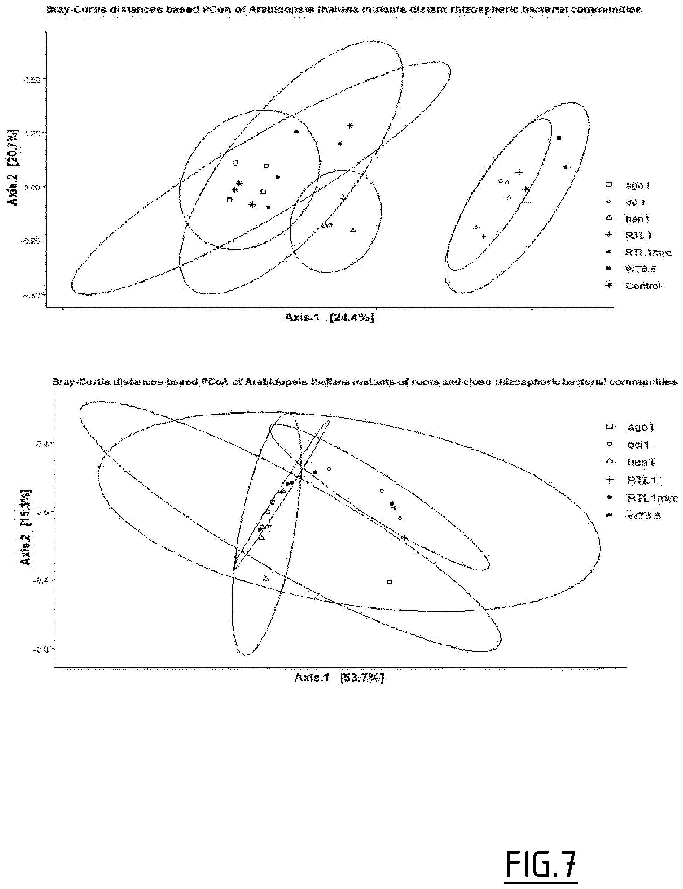

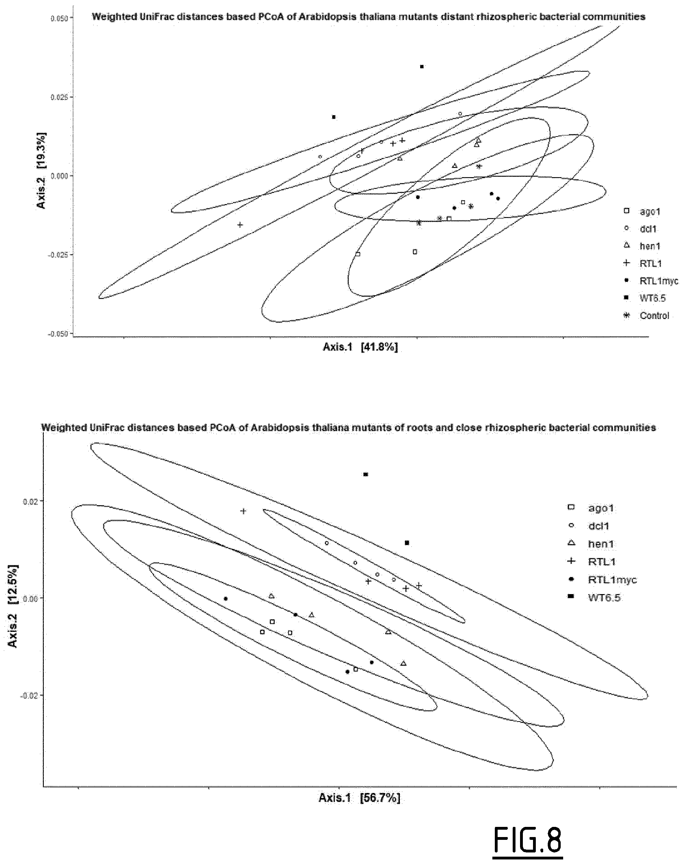

The Bray-Curtis distance ordination, which is based solely on ASV abundance but not phylogeny, shows a clear distinction between mutant bacterial communities, as Type explained between 55% (p<0.001) and 44% of variance (p<0.01), for distant rhizosphere and for roots and close rhizosphere, respectively. *RTL1* and *dcl1-2* have similar bacterial communities and are close to WT communities, in both sample types **(****Figure 7****)**. This is interesting, knowing that these mutants were slightly more diverse than WT, but not evenly, suggesting a few dominant bacterial taxa. Aside from these putative dominant taxa, their bacterial communities could be quite similar to WT. The Weighted UniFrac ordination **(****Figure 8****)**, which combines ASV abundance and phylogeny, also depicted Type as an important factor in bacterial communities, as it explained 52% for distant rhizosphere samples (p<0,001) and 39% for roots and close rhizosphere (p<0.05). This PCoA shows the same tendency as Bray-Curtis PCoA: *RTL1* and *dcl1-2* have the most similar bacterial communities in comparison with WT, whereas *ago 1-27, RTL1myc* and Control are often clustered together. *hen1-4* mutants seem to be in the middle between these two main clusters. It is especially noteworthy that the distant rhizospheres of *ago1-27* and *RTL1myc* have very similar bacterial communities to Control unplanted soils, as *ago1-27* is completely deficient in siRNA and miRNA production: this could suggest that without these small RNAs bacterial communities in the rhizosphere are more similar to soils without a plant, than to a WT rhizosphere. Obviously, suppressing the whole small RNA pathway will have incidents on many biological reactions, not just the pure excretion of RNA into the rhizosphere, however it is interesting to note the direct or indirect effect of such a mutation and the impact on bacterial communities.

### Fungi

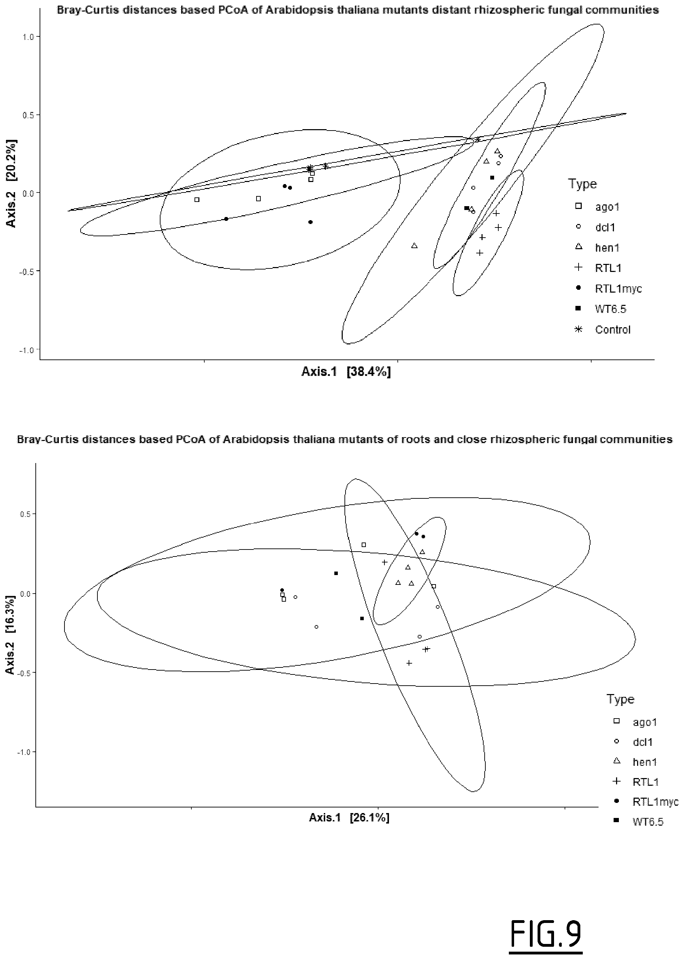

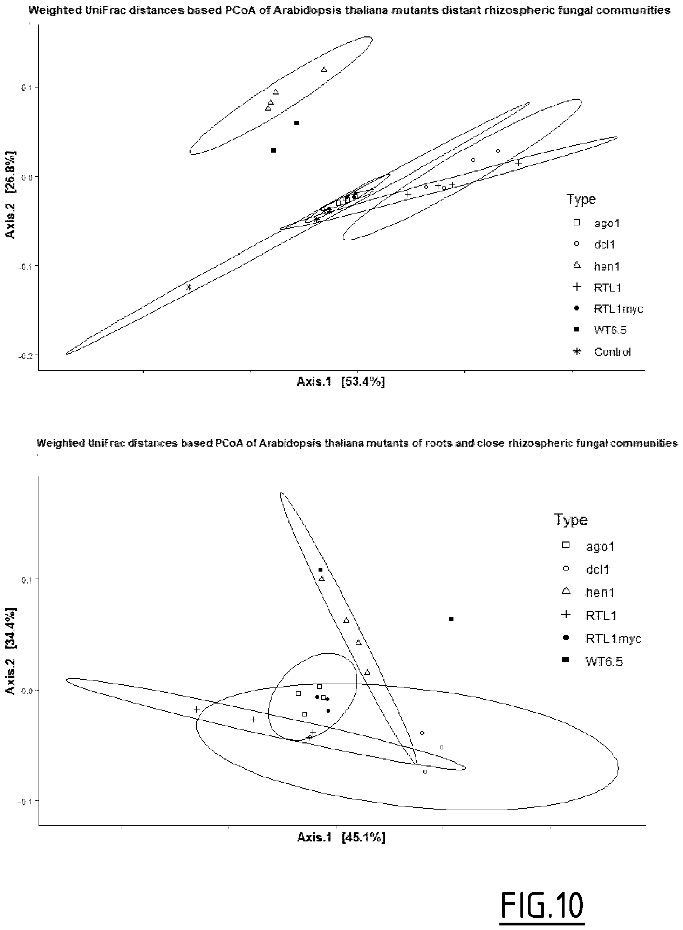

The Bray-Curtis ordination on fungal communities **(****Figure 9****)** reveals again the impact of Type 64% for distant rhizosphere samples (p<0.001) and 41% for roots and closer rhizosphere samples (p<0.01). The ordination for root samples does not display clear clusters. In the distant rhizosphere samples, the tendency is similar to what is seen in bacterial communities: a cluster with *RTL1myc, ago1-27* and Control and another cluster with *RTL1, dcl1-2* and WT. However, *hen1-4* seems closer to WT when regarding fungi. *RTL1myc* and *ago1-27,* in both fungi and bacteria, are quite similar. The Weighted UniFrac ordination **(****Figure 10****)** highlights these differences between fungal communities and Type explained between 61% and 76% of variance (p<0.001), for roots and close rhizosphere, and distant rhizosphere, respectively. In both sample types, the two major clusters discussed above are very clear, but WT fungal communities dissociate from the *RTL1* - *dcl1-2* cluster and have more commonalities with *hen1-4* mutants. These mutants have issues with siRNA and miRNA accumulation, even though it is discussed if non-methylated miRNAs are all destined to decay or not, and have similar fungal communities as WT: this could suggest that small RNAs do not interfere with fungal communities' assembly and dynamics, but why don't *ago 1-27* mutants also have similar communities? This could be due to the fact that *ago1-27* mutants do not form functional RISCs (RNA Induced Silencing Complex), so their miRNAs are not trimmed nor uridylated: they are not active, but they still could be potentially exported into the rhizosphere, for example. It is also noteworthy that some families of miRNAs are not affected by the lack of methylation in *hen1-4* mutants and show no noticeable modification at all. Maybe the miRNAs not affected suffice to create a "normal" enough environment for fungi.

### 4) Specific differential abundances and indicator species

Differentially abundant ASVs were investigated in comparison with WT, with very stringent parameters for bacterial taxa (p<0.001, top 50 most signif.) because of the large number of ASVs, whereas only a couple fungal ASVs were considered differentially abundant (p<0.001). Interestingly, in the distant rhizosphere, out of the top 50, only between 2 and 9 ASVs were enriched compared to WT, except for hen1-4 which had 23 enriched ASVs, whereas, in roots and close rhizosphere, 10-22 ASVs were enriched. Regarding the most differentially abundant bacterial ASVs, mutants are depleted in a majority of them compared to WT, especially in the distant rhizosphere. One could hypothesize that these plants have difficulties recruiting these essential bacteria, found in WT plants, due to siRNA and miRNA pathways disruption. Interestingly, *dcl1-2* and *hen1-4* roots and close rhizosphere were highly enriched in an ASV of the Massilia genus, which has been shown to be one of the most abundant and selected genera throughout *Arabidopsis* generations in wild-type plants. The control soil was enriched in ASVs from the phyla Proteobacteria and Planctomyces.

This could be extended to fungi too, as the majority of fungal differentially abundant ASVs were depleted in roots and rhizosphere. Interestingly, ASVs from the genus Clitopilus (Basidiomycota), were depleted in all mutants in the distant rhizosphere, including control soil, compared to WT, whereas they were enriched in roots and close rhizosphere samples.

Indicator species are ASVs that when present in a sample can give great information on the sample's nature, it is a species that is indicative of its environment. WT roots and rhizosphere can both be indicated by ASV 166 (p<0.01), which is related to the Massilia genus, a very commonly selected genus by *Arabidopsis* plants, as discussed previously. The Massilia ASV enriched in roots of *dcl1-2* and *hen1-4* must be different to ASV 166, indicator of WT: however, these mutants have succeeded in strongly selecting a species from an important genus, more than WT, maybe showing that these mutants could still select essential bacteria, but not specifically the same species.

Four fungal ASVs were indicative of *RTL 1* mutants in the distant rhizosphere: an ASV of the Olpidium genus (phylum: Chytridiomycota), an ASV from the Rhizopus genus (phylum: Zygomycota), one from the Clitopilus genus and one from the Solicoccozyma genus (both, phylum: Basidiomycota). The ASV "48", a Basidiomycota, was the only indicator species in root samples, indicative of WT samples.

In distant rhizosphere samples, WT plants have a great number of bacterial indicator species: these ASVs could be representative of a "normal" microbiota, species that have not been able to settle in the rhizosphere of mutants, which are maybe more similar between them. It should be noted that WT samples have beneficial indicator species such as ASVs from the Herbaspirillum genus (endophyte, nitrogen-fixing) and the Pseudomonas genus (plant growth and health promoter): no other mutant has indicator species from these important genera. Interestingly, in both sample types, *hen1-4* has a great number of significant bacterial indicator species, suggesting that these bacteria are near specific to this mutant. This can be verified on the Bray-Curtis ordination **(****Figure 7****)** as *hen1-4* bacterial communities are clustered alone, in distant rhizosphere samples.

Interestingly, the microbial communities in distant rhizosphere seem perhaps more affected by the plant's mutation than the communities directly in the roots or very close to them. Even though the tendencies are similar, the differences between communities are often more visible in distant rhizosphere samples, in our PCoA figures with both distances. To conclude, these initial analyses show that:
1. Rhizospheric microbial communities are not unaffected by the disruption of plant miRNA and/or siRNA production,
2. Communities from mutants having only miRNA or only siRNA pathway affected are more similar and closer to wild type communities. Hence *RTL1* and *dcl1.2* mutants are often clustered near wild type samples, but in fungal communities *hen1.4* are also close to wild type, suggesting this mutation may affect more bacterial than fungal species. Thus one could hypothesis the existence of a compensatory mechanism in mutants *RTL1* and *dcl1.2.*

### Manipulation of miRNA159c by miPEP159c treatment: impact on plant tolerance to abiotic stress and on the rhizomicrobiome

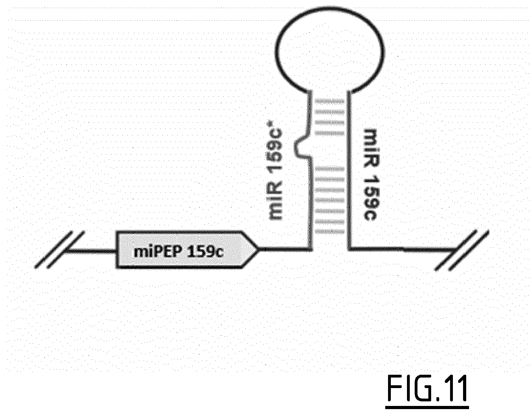

In order to investigate more precisely the role of miRNAs in the rhizosphere and on the microbial communities present, our study focused on one specific miRNA family: miRNA 159. It is one of the most important miRNA families in plants: they are very well conserved and abundant in many plant species, making them a nice study model. Moreover, four out of six miRNAs found in common in the rhizosphere of *B. dystachion* and *A. thaliana* were from the miRNA 159 family. To study the effect of these miRNAs, miRNA encoded peptides (miPEPs, see **Figure 11****)** were used to specifically enhance the transcription of certain members of the miRNA family. In view of the members of the "core miRNA" found in the rhizosphere scanning experiment, our focus remained on the miRNA1 59a, miRNA1 59b and miRNA1 59c, which were over-expressed due to miPEP159a, miPEP159b and miPEP159c, respectively.

### Phenotypic changes after miPEP treatment

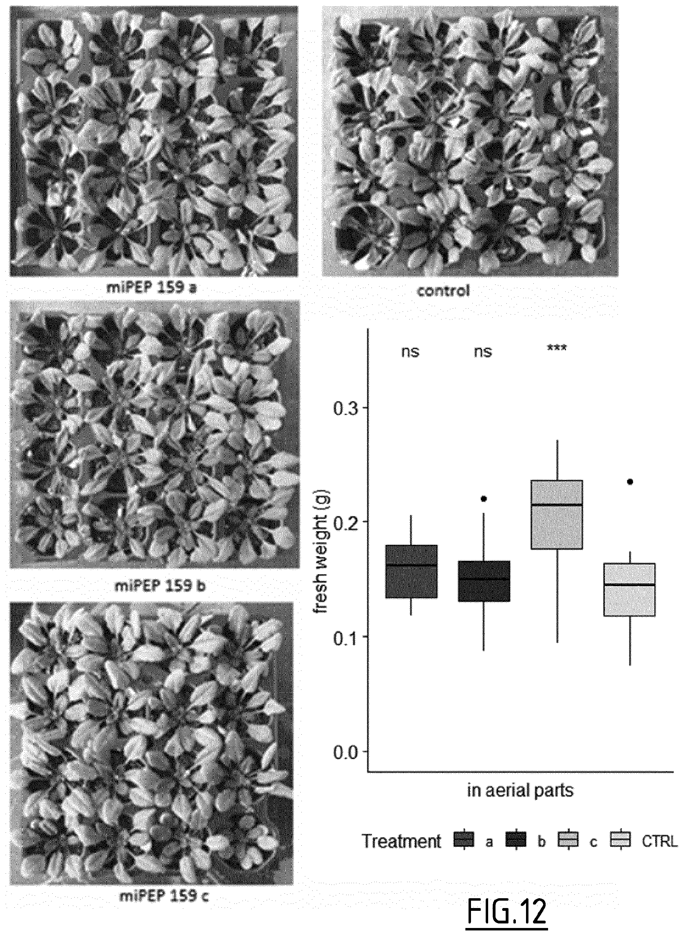

As described in the Material and Methods, plants were only watered with 500µL of water + miPEP, three times a week, this induced an unexpected water stress in the plants. On the third week of treatment, the leaves were clearly showing lack of hydration, except for plants treated with miPEP159c **(****Figure 12****).** Indeed, the fresh weight of leaves, and their water content, from plants treated with miPEP159c were very significantly higher than the control plants (Wilcoxon test: p<0.001). The dry weight of the aerial system showed no differences between treatments (data not shown), indicating that miPEP159c plants were more hydrated, either by better retaining water or by taking-up water more easily. No differences were seen in rosette diameter or number of leaves (data not shown), between miPEP159c plants and control.

Plants treated with miPEP159c seem to have a better tolerance to water stress: this experiment was repeated and the results were very similar: leaves from plants treated with miPEP159c had significantly higher fresh weight. Interestingly, the plants treated with miPEP159c were not as healthy looking as in the first experiment, however, their leaves had a purple coloration, not seen in other treated plants. This could suggest a higher amount of anthocyanins produced in these miPEP159c treated plants: these pigments are known for having protective effects in plants, especially against abiotic stresses. In this second experiment, a "scrambled" miPEP159c was used: the amino-acids are the same as in miPEP159c but in a random order. Plants treated with this scrambled peptide did not show the same effects as the ones treated with miPEP159c, suggesting that the mechanism by which this treatment works is not simply due to its peptidic composition, but is clearly by a subtler mechanism, such as activating specifically the transcription of its associated miRNA.

### Rhizospheric microbial communities of miPEP-treated plants

The aim of this experiment was to see if the miPEP treatment, *i.e.* the manipulation of a specific miRNA, affected the rhizospheric microbial communities. To answer this question, DNA from roots and rhizosphere of treated plants, but also treated unplanted soils, was extracted and sent for sequencing. The Control-mi-PEP samples correspond to plants and soils treated with only water, with no solubilized miPEP. The nature of the samples: roots and close rhizosphere; distant rhizosphere and unplanted soils are as described for the mutant experiment. The following results originate from the combination of 16S and ITS sequencing and quantification by qPCR.

### 1) General composition at the phylum and genus level

### Bacteria/Archaea

As expected, all samples, in all conditions, are dominated by Proteobacteria, followed by Bacteroidetes and Verrucomicrobia in root samples, and Bacteroidetes and Actinobacteria in soil samples. Planctomycetes were also quite abundant in all samples. These bacterial phyla are very common in the roots and rhizosphere of Angiosperms, which are both dominated by Proteobacteria followed by varying proportions of other phyla. Due to quantitative measures, by qPCR, root and close rhizosphere samples have the highest amount of bacteria sequenced, which englobes endophytes, rhizoplane-living bacteria and close rhizosphere-living bacteria. Distant rhizosphere and unplanted soils are quantitatively similar. DNA from root samples and soils was not extracted using the same method, but the same quantity of DNA was used for all samples during qPCRs. Aside from the expected differences in phylum composition between sample types (roots VS soils), no great differences are observed between miPEP treatments. As for the main bacterial genera, root samples are dominated by the Rhizobacterium genus (Proteobacteria) and Opitutus genus (Verrucomicrobia), and in smaller portions: Devosia, Dongia, Rhodanobacter, uncultured-Chitinophagaceae, Planctomyces, Pseudolabrys and Blrii41 FA genera. Distant rhizospheres and unplanted soils were also dominated by Rhizomicrobium, followed by Planctomyces and Pseudolabrys. Unlike the root samples, the Luteimonas genus (Proteobacteria) was in the soil samples' top phyla. The Opitutus genus was not in the top phyla in unplanted soils, compared to other sample types. The general information in these compositional analyses show that no important microbiota changes are seen between miPEP treatments.

### Fungi

On the fungal side, soil samples and especially unplanted soils had higher quantities of fungal copies. Interestingly, unplanted soils treated with miPEPs have more fungal copies than the control unplanted soils. This could be due to the nutritive value of miPEPs for some fungi. Root samples are mainly composed of Basidiomycota and Ascomycota, followed by Zygomycota and Chytridiomycota, and in smaller proportions Glomeromycota and Neocallimastigomycota. The Glomeromycota phylum is essential to plants, as members form arbuscular mycorrhiza with over 80% of plants world-wide: these symbionts help with plant nutrition and health. The Neocallimastigomycota phylum is composed of one family of which members are anaerobic and found in the gut of herbivores, in which they degrade plant materials. These fungi could have originated from the potted soil used in the experiment. The same phyla were found in soil samples, with higher proportions of Chytridiomycota, especially in distant rhizospheres. No great difference of fungal phyla composition between treatments is shown, for more precisions, the top genera were analysed. Root samples were dominated by Solicoccozyma and Cladosporium genera: it is interesting to note that plants treated with miPEP159c had a greater proportion of Cladosporium and Triparticalcar generea. Cladosporium are fungi generally nonpathogenic, found in soils and plants: some species can be phytopathogens, but some are also considered endophytes or phylloplane fungi. For example, *Cladosporium cladosporioides* was abundantly found in halophytes of the Red Sea, however, they were described as "common non-specific saprobes", unrelated to the salty environment. Triparticalcar is a small genus (2 species) of soil-inhabiting coprophilous fungi. Compared with roots, soil samples have a higher proportion of Triparticalcar, especially in distant rhizosphere, in which they are the dominating genus. One noticeable difference between soil samples is the higher quantity of Candida genus in unplanted soils. Otherwise, there are no striking differences between treatments.

This global approach of looking at the main bacterial and fungal phyla and genera found in our samples does not reveal any drastic microbiota changes. Either the over-expression of one specific miRNA, by miPEP application, has no impact on the composition of the root and rhizospheric microbial communities of water-stressed *A. thaliana,* or the changes are at a smaller, more specific level, such as the species level (see enriched/depleted species and indicator species).

### 2) Assessing diversity within samples (alpha-diversity)

After checking the general composition of our samples, the diversity within them was analysed, with alpha-diversity indices.

### Bacteria/Archaea

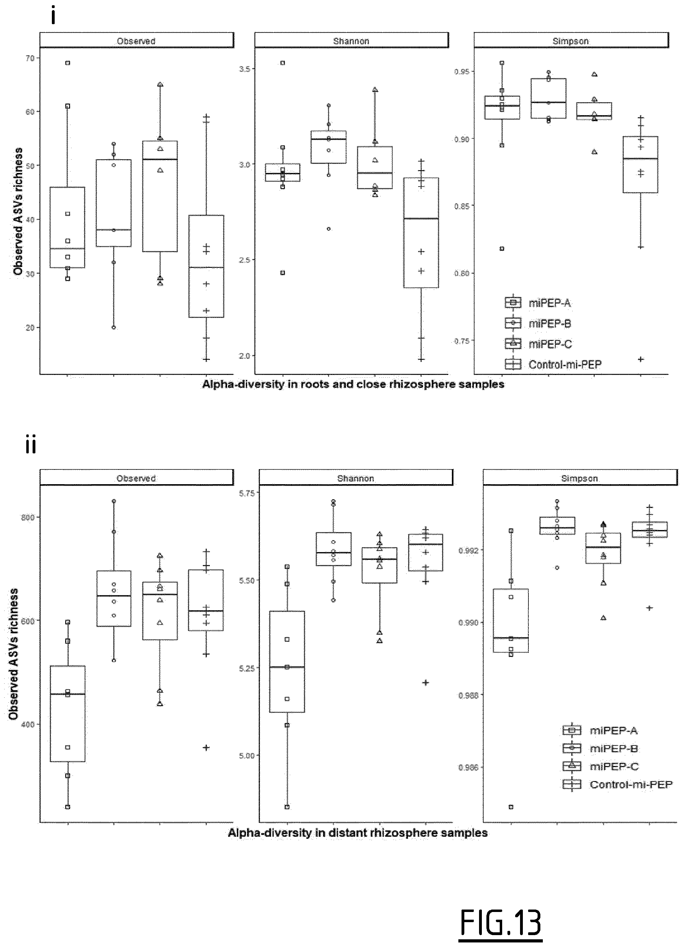

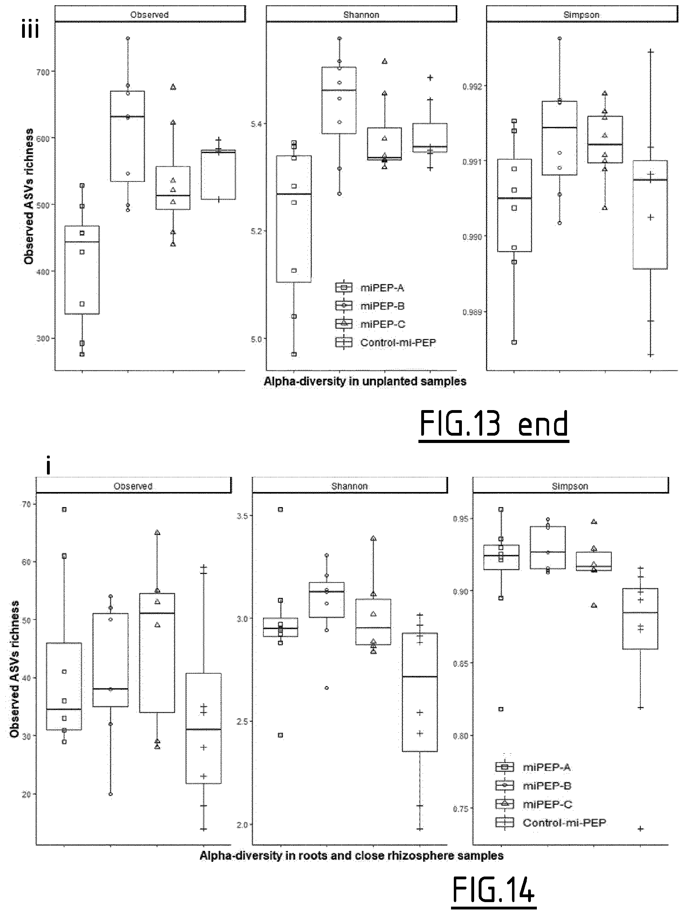

Prior to the analysis, roots samples, distant rhizosphere and unplanted soil samples were rarefied at, respectively, 4 614 100 copies, 588 824 copies and 323 104 copies. In root and close rhizosphere samples, plants treated with miPEP-B or miPEP-C had slightly more observed bacterial species than Control (p<0.1) **(****Figure 13****).** miPEP-C treated plants also had a slightly higher Shannon index (p<0.1). Interestingly, distant rhizospheres from miPEP-A treated plants were very significantly less diverse than Control (observed species p<0.01; Shannon p<0.001; Simpson p<0.001). This was also the case in unplanted soils (observed species p<0.01; Shannon p<0.01). This may suggest that miRNA159a, when overexpressed, has a negative impact on bacterial soil diversity.

### Fungi

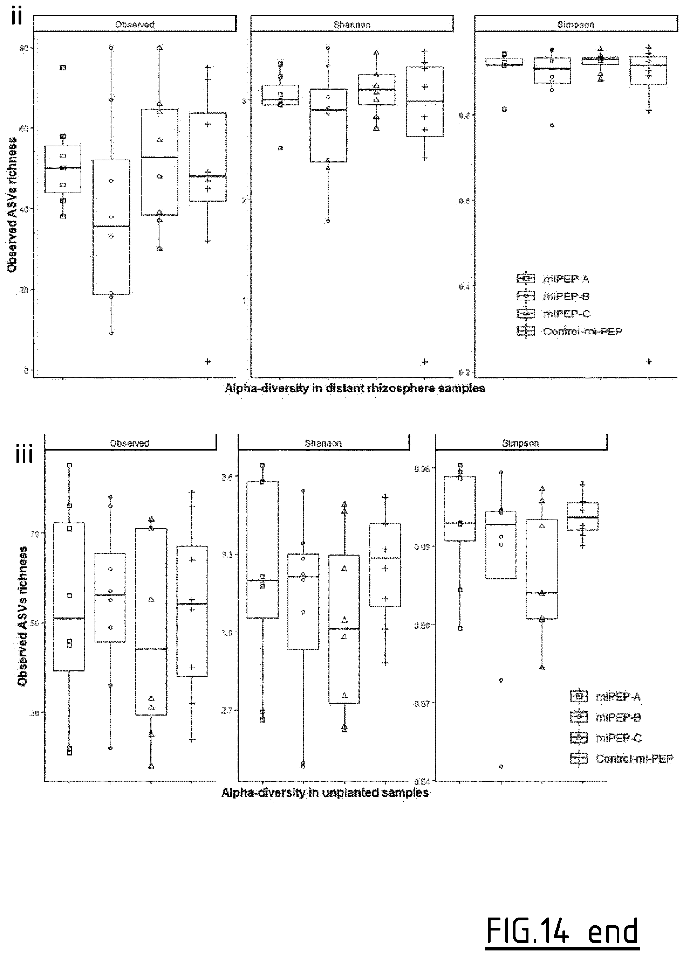

Prior to the analysis, roots samples, distant rhizosphere and unplanted soil samples were rarefied at, respectively, 31 281 copies, 1 254 140 copies and 502 744 copies. In root and close rhizosphere samples, plants treated with miPEPs had significantly higher Shannon and Simpson indices than Control, but not of observed species **(****Figure 14****).** This may be due to the nutritive aspect of the miPEP solution, as a source of carbon and nitrogen, allowing a larger group of fungi to survive. In both soil sample types, no significant difference in alpha-diversity between treatments was observed.

### 3) Estimating the differences between mutants (beta-diversity)

To investigate more deeply the composition and similarities between communities, beta-diversity was estimated throughout our samples, using both Bray-Curtis and Weighted UniFrac distances.

### Bacteria/Archaea

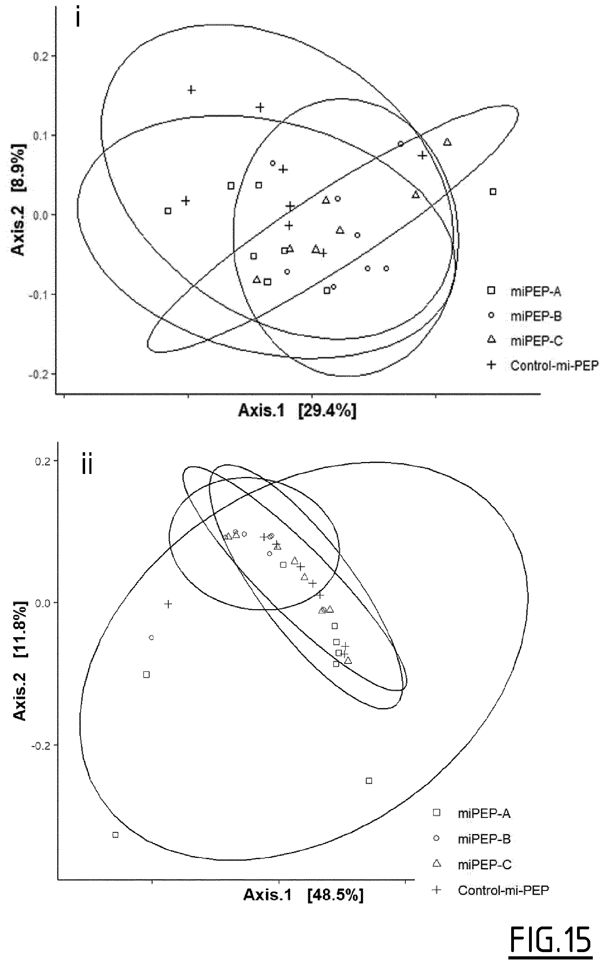

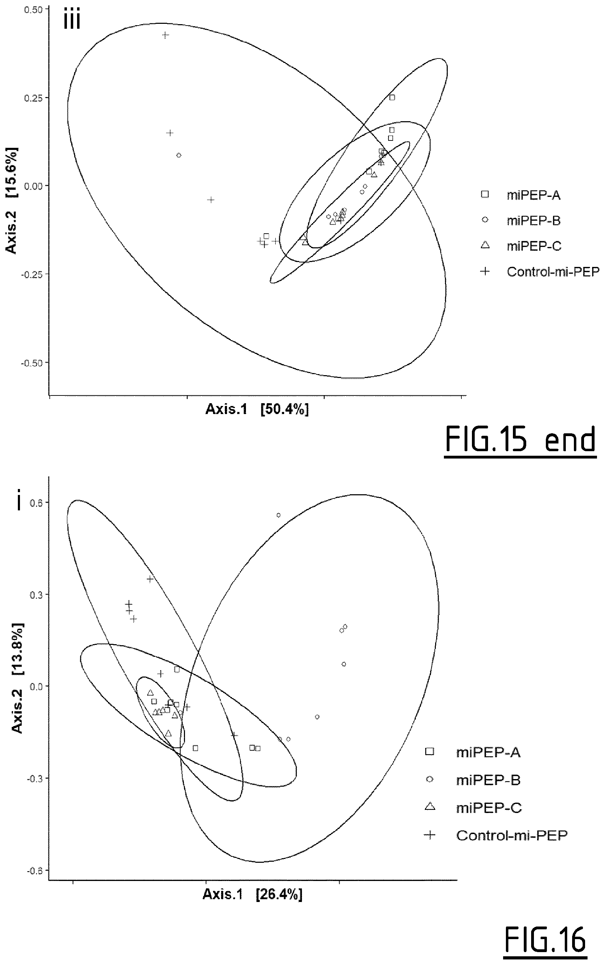

Bray-curtis ordination of bacterial communities in root and close rhizosphere samples shows no clear clustering **(****Figure 15****),** even though miPEP treatment counted for -14% of variation (p<0.1). No clear distinction was seen in distant rhizosphere samples either, though treatment explained -15% of variation (p<0.1). Concerning the unplanted samples, the miPEP-treated soils are clustered together apart from the Control samples: this explained near 25% of variation (p<0.01). Maybe the miPEP solution served as a nutritive source, as miPEPs are quickly broken down in soils, and helped different bacteria grow than those found in the Control group, explaining this clustering.

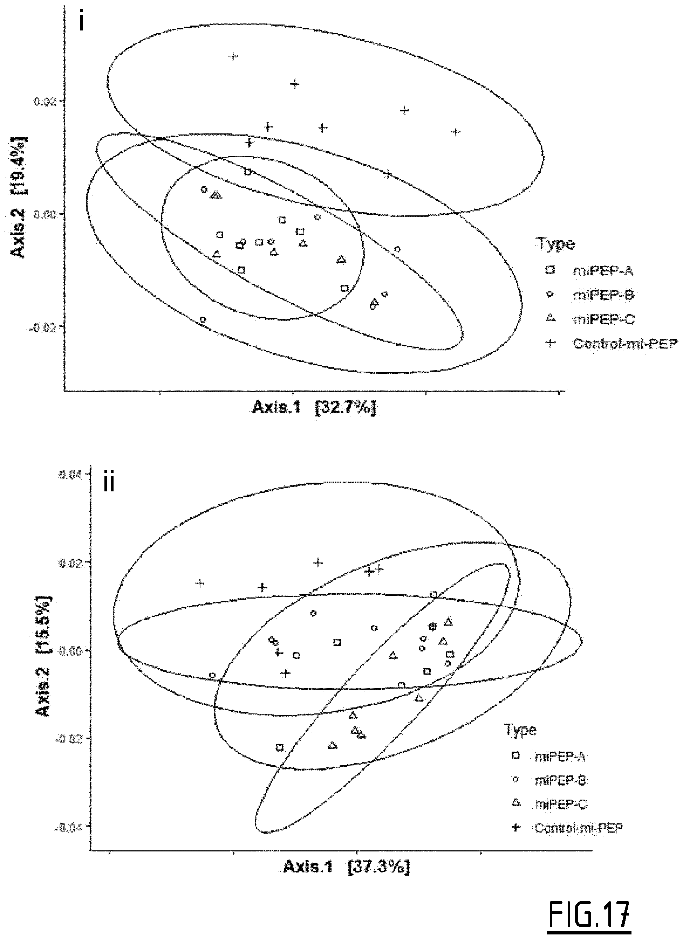

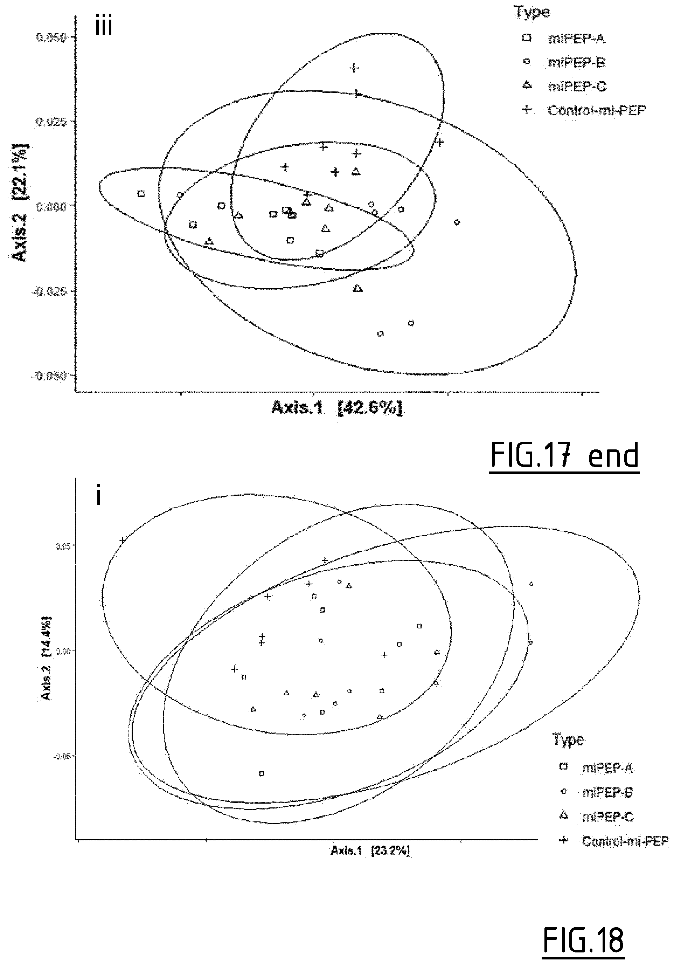

Weighted UniFrac distances, which take into account phylogeny and abundance, show that bacterial communities of Control samples are different to those of miPEP-treated root samples **(****Figure 17****):** this treatment explains 22% of variation (p<0.01). Once again, this could be due to the nutritive aspect of miPEPs. This clustering also appears in distant rhizosphere samples in which treatment counts for only 24% of variation (p<0.001). In unplanted samples, treatment explained up to 32% of variation (p<0.001), but the Control cluster was not as distant from the others, as in Bray-Curtis ordination.

It is interesting to note that these two ordination types do not show the same patterns, where Bray-Curtis ordination gives an idea of the species and their abundance shared between samples, Weighted UniFrac distances are calculated based on the divergence between sequences in each sample. This means that if two communities have completely different species, but that these species are close phylogenetically, the two communities will be considered less different, than with a matrix that does not take into account phylogeny. With this ordination method, bacterial communities between Control and miPEP-treated roots are actually quite different. The fact that all miPEPs are clustered together makes the hypothesis of the nutritive source more plausible than actual miPEP action. Unless miRNAs from the same family have the exact same impact on bacterial communities.

### Fungi

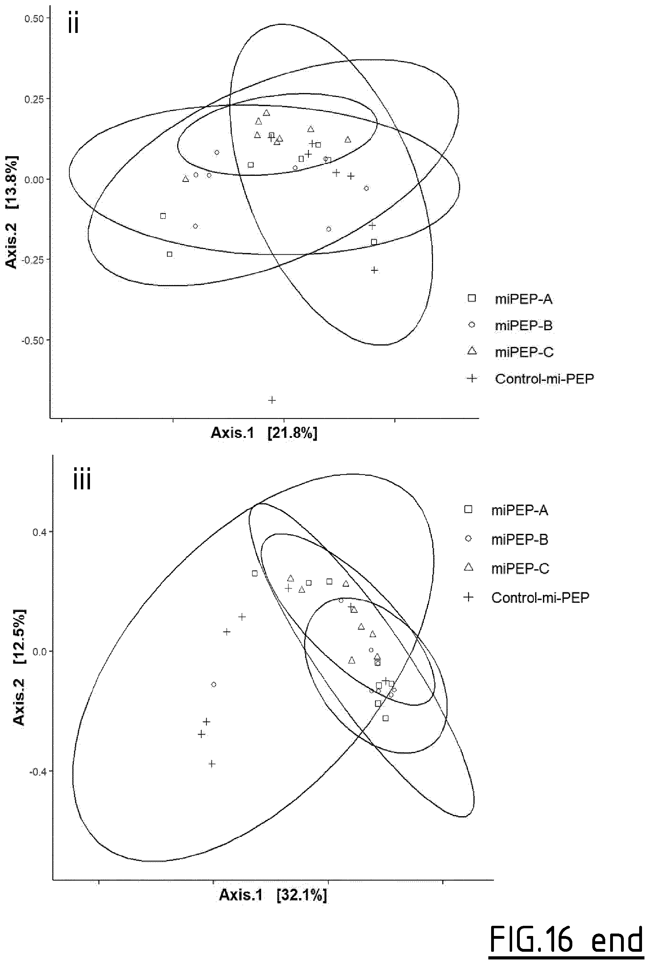

Visually, Bray-Curtis ordination showed better clustering for fungal communities **(****Figure 16****)**: however, treatment only explained for near 29% of variation (p<0.001) in root samples. In soil samples, clusters were less clear and treatment explained 18% (p<0.01) of variation in distant rhizosphere samples, however the dispersion test (p<0.01) showed that this variation was more due to group dispersion than to biological effect. Type explained for 19% (p<0.01) of variation in unplanted samples. However, Control communities clustered together far from miPEP-treated unplanted soils (and to some extent in distant rhizospheres), similarly to bacterial communities in unplanted soils.

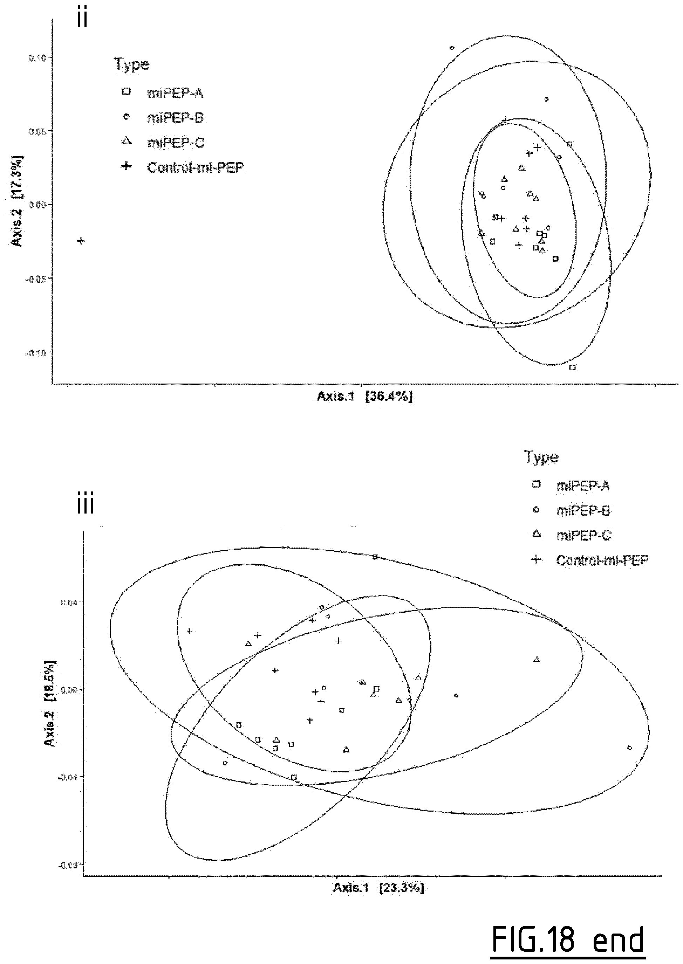

Weighted UniFrac ordination shows subtle clusters for fungal root samples (14% variation, p<0.05), but not in distant rhizosphere samples (12% variation, non-significant), nor unplanted samples (14%, p<0.05) **(****Figure 18****)**.

The fact that the fungal communities are more distinct between treatments with Bray-Curtis than with Weighted UniFrac could mean that the fungi that these communities share are actually closely related, but not exactly the same species. This is interesting as we have already discussed that the differences between treatments is not on a global scale (top phyla, top genera), but probably at a more specific level.

### 4) Specific differential abundances and indicator species

### Bacteria/Archaea

The DESeq2 package was used to evaluate the presence of significantly enriched/depleted ASVs, compared to Control, in our conditions. Very stringent parameters were applied to analyse the differentially abundant ASVs: only top 50, highly significant (p<0.001), ASVs were regarded. Root samples treated with miPEP-B or miPEP-C were highly enriched in a species of Bryobacter, a genus found in Sphagnum mosses in acidic wetlands, and in species of Burkholderia-Paraburkholderia and Streptomyces, which of some species are plant beneficial bacteria. miPEP-treated samples all were enriched, in comparison with Control, in 2-3 species of Rhizobium, a well-known plant growth promoting bacteria genus and known for their symbiotic relationship with Fabaceae plants. P30-42FA is a genus within the Myxococcales order, also known as "slime bacteria", often found in soils and decomposing plants, are significantly enriched in miPEP-C treated plants. In distant rhizosphere, miPEP-C treated plants are most enriched in a species from the Acidothermus genus, however it only contains a unique species, found in extreme environments, with an optimal growth temperature at 55°C. However, it is closely related to the mesophilic actinobacteria *Frankia* genus, which is known for nitrogen-fixing and living in nodules in plant roots. A species from the Tepidisphaera genus (Planctomyces), which are moderate thermophiles, was the most enriched in miPEP-B or miPEP-C treated unplanted soils.

Indicator species are specific ASVs that are indicative of a certain type of environment, or in our case: miPEP-treatment. The most significant indicator species in Control root samples was ASV 116, from the Phenylobacterium genus (p<0.001), followed by ASV 1083 from the Opitutus genus and ASV 614 from the Burkholderia-Paraburkholderia (both, p<0.01). Interestingly, *P. immobile* is the only species of Phenylobacterium genus, which only grows on very limited nutritive sources, such as specific herbicides. In root samples, ASVs 1677 (HTA4OR, Proteobacteria), 439 (uncultured-Chitinophagaceae, Bacteroidetes), 1958 (TepidisphaeraceaeFA, Planctomycetes) and 652 (Pirellula, Planctomycetes) were indicative of miPEP-B treated plants (p<0.01); ASV 417 (uncultured-Chitinophagaceae, Bacteroidetes) (p<0.001), ASV 323 (Rhodopseudomonas, Proteobacteria), ASV 1702 (Rhizomicrobium, Proteobacteria) and ASV 1642 (Flavobacterium, Bacteroidetes) were indicative of miPEP-C treated plants (p<0.01).

In soil samples, ASVs 889 (Variibacter, Proteobacteria), 327 (Haliangium, Proteobacteria) and 406 (Steroidobacter, Proteobacteria) were very significantly indicative of Control samples (p<0.001). ASV 325 was very significantly indicative of miPEP-C treated plants' distant rhizosphere (Caenimonas, Proteobacteria) (p<0.001), species from the genus are often isolated from soils and are related to the Burkholderiales order, which contains many plant growth promoting bacteria. There were +10 ASVs highly significant indicator species in unplanted soils treated with miPEP-A and miPEP-B, compared with Control and miPEP-C treated soils.

### Fungi

Root samples from miPEP-treated plants were all enriched with species from Solicoccozyma (basidiomycetous yeasts), Hyaloscypha (saprobic, lives on dead wood) and Candida (common yeast), compared with Control plants. Root samples treated with miPEP-B or miPEP-C are enriched with a fungus from the Pandora genus, which is the most common fungal insect pathogen, often found in aphids. Interestingly, the distant rhizospheres of miPEP-B or miPEP-C treated plants were enriched with Aspergillus species: some of which are known for being pathogens such as *A. flavus,* but also beneficial species such as *A. japonicus* which improved plant biomass and growth under heat conditions and other plant growth promoting species, such as *A. ustus* and *A. terreus.* In unplanted soils, samples treated with miPEP-A or miPEP-C were enriched with an ASV from the Paraconiothyrium genus, which is very common in soils. One treated with miPEP-B or miPEP-C were enriched with Pseudogymnoascus genus, which are found in soils and some species are capable of mycorrhiza with certain plants (Ericaceae family).

Interestingly, root samples from plants treated with miPEP-C had the most significant (p<0.001) differentially abundant ASVs, both bacterial and fungal, compared with Control.

Three fungal ASVs were significantly indicative of plants miPEP-C treated plants, in root samples: ASV 390 (Bambusaria, Ascomycota), 121 (Cladosporium, Ascomycota), and 7 (Triparticalcar, Chytridiomycota) (p<0.01), these last two genera were found in greater proportions in miPEP-C samples. As seen previously, Cladosporium species are often found on plants, notably on leaves, and they are resistant to changing water contents in their environment, many of these species are also halotolerant, like *C. sphaerospermum,* which has been found in plants. Multiple studies have already shown the potential for halotolerant bacteria and fungi to help plants grow in stressful conditions, such as water stress. Triparticalacar fungi are part of the Spizellomycetales family and can be both beneficial or harmful to plants, as they can parasite a number of organisms: some damageable, like nematodes, but also beneficial organisms, like AMFs.

Four ASVs were indicative of Control in root samples: two non-assigned, a Solicoccozyma species and a Hyaloscypha species. In the distant rhizosphere, the most significantly enriched ASV was ASV 72 (Aspergillus, Ascomycota) in miPEP-C treated plants (p<0.001), as noted previously this could well be a beneficial Aspergillus species. In unplanted soils, a few species were indicative of miPEP-A or miPEP-B treatments but not the other types.

To conclude on the effect of miPEP159 on the rhizospheric microbiota, it is clear that the over-expression of one specific miRNA does not drastically change the microbial communities in the plant's rhizosphere. The microbial differences between treatments were at the species level, suggesting a very specific effect that miPEP treatments may have on a microbial community. However, these minor changes in the microbiota may have a great impact on the plant's fitness as there was a significant beneficial effect of miPEP159c on *A. thaliana's* tolerance to water stress.

### Extracellular vesicle production is highly active in rhizodermis root cells

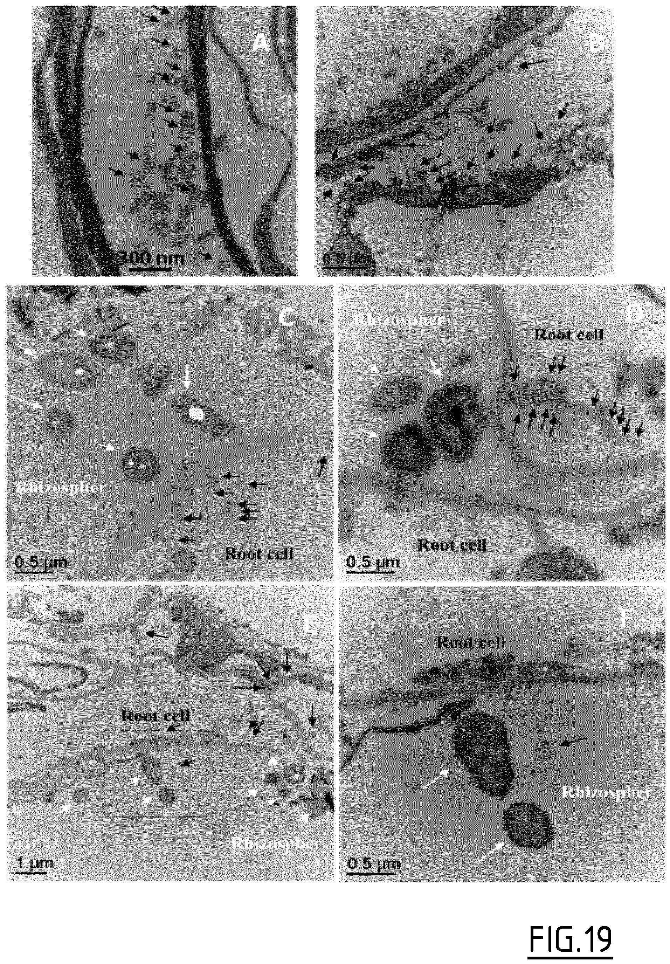

A transmission electron microscopy (TEM) analysis was performed to investigate the potential excretion of extracellular vesicles (EVs), as well as a possible exchange of EVs between root and rhizospheric microorganisms. Our observations of chemically fixed *A. thaliana* root samples revealed a large number of EVs in the rhizodermis cells **(****Figure 19, A****),** as well as a wide variety of EV sizes. Plants grown on agar also show this accumulation of EVs in their rhizodermal cells **(****Figure 19, B****).** The samples from plants that have grown on soil **(****Figure 19****, C and D)** show an accumulation of these vesicles very close to the plasmalemma of rhizodermal cells, at the vicinity of bacteria. These observations suggest that root cells synthesize a high number of vesicles and possibly exchange these membranous structures with soil bacteria. Interestingly, vesicles are also found in the rhizosphere and colocalize with bacteria **(****Figure 19, E****).** Thus, these encouraging observations showing the presence of exosomal structures in the rhizosphere allowed us to establish a protocol for exosomal extraction. In parallel, and in order to complete our previous observation, cryo-fixed samples were also used. Indeed, unlike the chemical fixation technique, cryo-fixation allows observation without disturbing structures, which gives an idea of the organization *in situ.* The method of chemical fixation involves numerous washes and baths which can damage the structural association between root and attached microbes. High pressure and freezing (HPF) microscopy avoid any possible degradation and allow an observation of the rhizosphere without any structural perturbation.

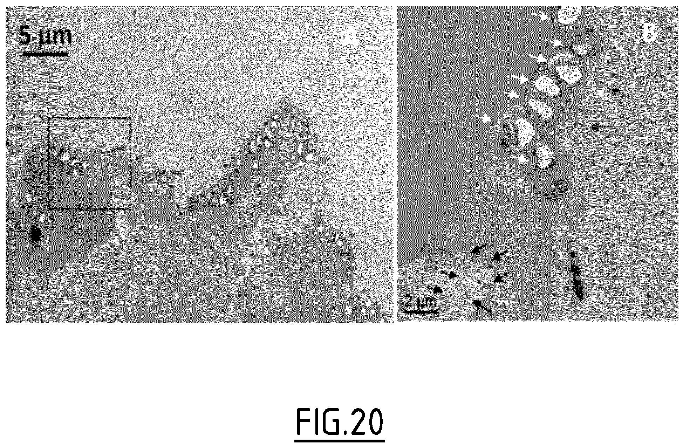

In roots grown in the soil, we found a large number of bacteria directly attached to the root. We still see vesicles inside the plant **(****Figure 20, B****).** However, bacteria have a different conformation to those observed by chemical fixation. Bacteria seem to be agglomerated in a biofilm (dark grey arrow) suggesting a close structural association between root surface and the rhizomicrobiome promoting a possible exchange of exosomal structures.

### Purification of rhizosphere root secreted exosome

Transmission Electron Microscopy (TEM) observations confirm the presence of vesicles in the rhizosphere and confirm the interest of setting up a protocol for extracting these extracellular structures. Many protocols allowing extraction or isolation of vesicles have been identified in the literature. Nevertheless, the protocols described are mainly suitable for extracting exosomes from animals, because they are much more studied for their implication in medicine as a bioindicator of disease or as a transporter of molecules of pharmacological interest.

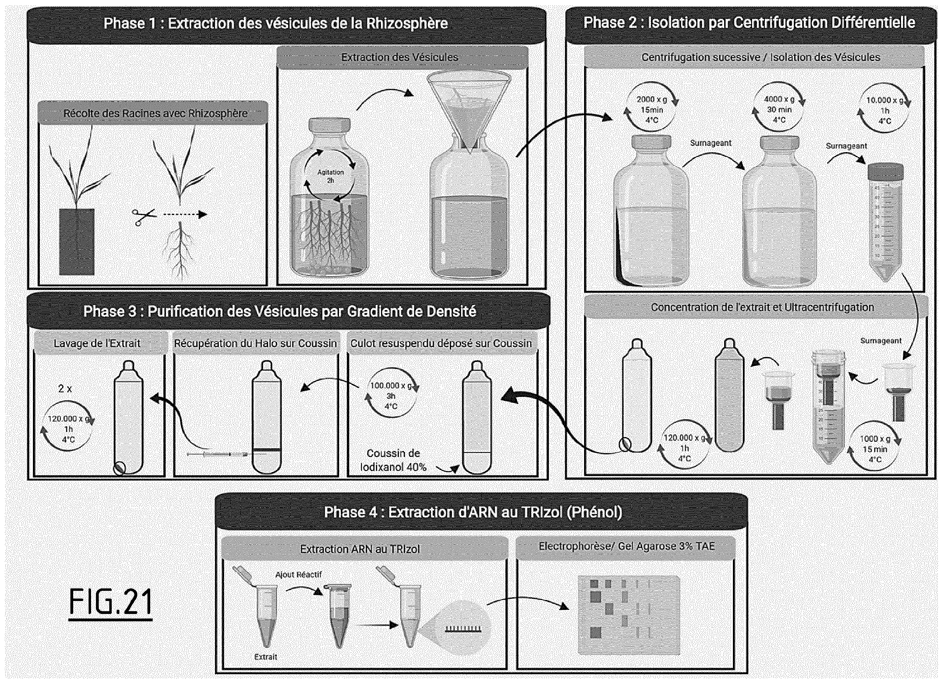

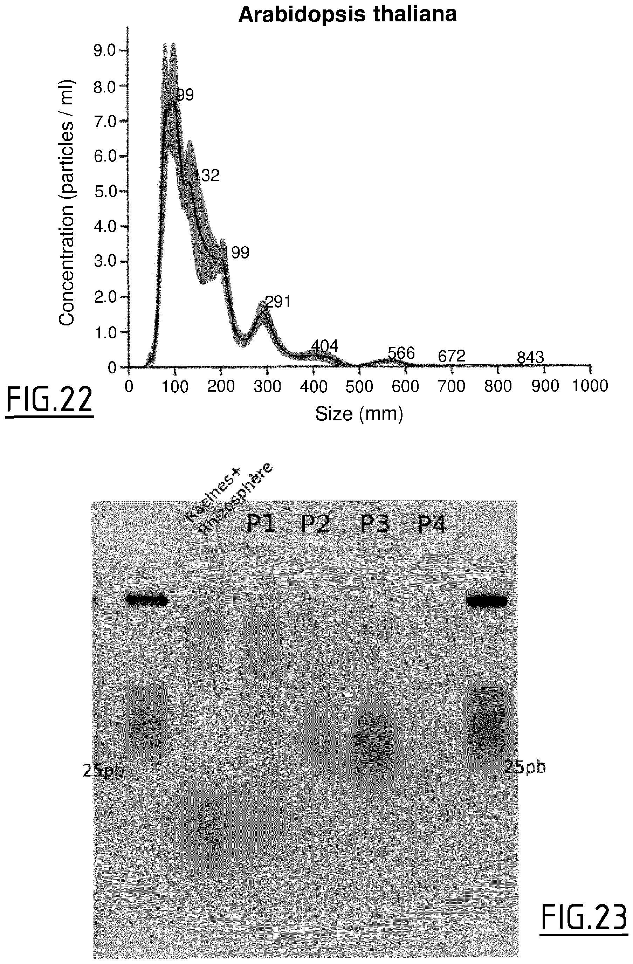

To extract the exosomes from the rhizosphere, we use a protocol based on differential centrifugations. As indicated in materials and methods, after filtration of large debris, we set up three steps of 2000g, 4000g and 10,000g centrifugation, followed by concentration on an Amicon^{®} column, followed with an ultra-centrifugation step **(****Figure 21****).** To estimate the quality and quantity of the exosomes, a Bradford assay was performed followed by an analysis of the extracts by NTA (Nanoparticle Tracking Analysis). NTA allowed us to determine the quantity of extracted vesicles: the results obtained indicate high concentrations of vesicles, between 1011 and 1012 particles per mL, as well as a distribution of their sizes in agreement with our predictions since the average size is around 100 -200 nm **(****Figure** 22). The Bradford assay is a classic approach used to estimate vesicle concentrations, by measuring protein concentrations, which become higher with purification (**see table 2**). To follow the evolution of the RNA profile according to the different steps of the protocol, samples from three steps were used: the first one from the filtrate after filtration on gauze (P1), the second after concentration on an Amicon^{®} column (P2) and the third on the pellet obtained at 120,000 g (P3).

**Table 2: Protein concentration measured from samples taken at each purification step**

| Purification steps | Protein concentration mg/ml |
|---|---|
| P1 | 0.15 |
| P2 | 0.28 |
| P3 | 0.24 |

RNA was extracted and deposited on a gel. The results of the RNA profile show the evolution of the RNA content at different stages of the protocol. This exosome extraction protocol gradually allows the elimination of large RNAs greater than 100 bp (**Figure 23**). This progressive enrichment in RNA of small sizes, would indicate that the exosomes mainly contain small RNA.

## Claims

1. A method for selecting at least one micropeptide able to alter the microbiome influenced by roots of a plant, wherein said method comprises:
a) identifying (i) at least one miRNA secreted by said plant in a sample of the roots and/or rhizosphere of said plant or (ii) at least one miRNA secreted by said plant which is identical to a secreted miRNA of a control plant, wherein the secreted miRNA of the control plant is able to alter the microbiome influenced by roots of said control plant,
b) identifying at least one ORF associated to at least one miRNA identified in step a) and coding for a micropeptide,
c) providing at least one micropeptide encoded by one ORF identified in step b) and applying said micropeptide onto said plant,
d) comparing the microbiome influenced by roots of the plant in the presence or absence of said micropeptide, and
e) selecting at least one micropeptide able to alter the microbiome influenced by roots of the plant.

2. The method according to claim 1, wherein the micropeptide in step c) is obtained by chemical synthesis or by recombinant technology.

3. The method according to claim 1 or 2, wherein step d) comprises determining the genus and/or species of microorganisms and, optionally, their proportion, quantity and/or concentration, in a sample of the roots and/or rhizosphere of the plant in the presence of said micropeptide and in a sample of the roots and/or rhizosphere of the plant absence of said micropeptide.

4. A micropeptide, wherein said micropeptide is able to alter the microbiome influenced by roots of a plant.

5. The micropeptide according to claim 4, wherein said micropeptide is selected by the method according to any one of claims 1 to 3.

6. The micropeptide of claim 4 or 5, wherein said micropeptide comprises a sequence selected from the group consisting of sequence SEQ ID NO: 1, SEQ ID NO: 2 and SEQ ID NO: 3.

7. A composition comprising at least one micropeptide according to any one of claims 4 to 6.

8. A device suitable for spraying or watering, wherein said device comprising at least one micropeptide according to any one of claims 4 to 6 or a composition according to claim 7.

9. A method for altering the microbiome influenced by roots of a plant, comprising administering to said plant at least one micropeptide according to any one of claims 4 to 6.

10. The method according to claim 9, wherein said micropeptide is provided in the form of a composition according to claim 7 or in a device according to claim 8.

11. The method according to claim 9 or 10, wherein altering the microbiome of the plant induces a phenotype of interest in the plant.

12. Use of a micropeptide according to any one of claims 4 to 6 as a plant microbiome modulating agent.

13. The use according to claim 12, to induce a phenotype of interest in a plant.

14. The use according to claim 12 or 13, wherein said phenotype of interest is selected from the group consisting of a resistance to at least one plant pathogen, a resistance to at least one stress, or a resistance to at least one pollutant and their combinations.

15. The use according to any one of claims 12 to 14, wherein said micropeptide is provided in the form of a composition according to claim 7 or in a device according to claim 8.


SEQUENCE LISTING

<110>  UNIVERSITE DE RENNES 1
       CENTRE NATIONAL DE LA RECHERCHE SCIENTIFIQUE
       UNIVERSITE PAUL SABATIER TOULOUSE III
       INSTITUT NATIONAL DE LA RECHERCHE SCIENTIFIQUE (INRS)

<120>  METHODS FOR ALTERING THE MICROBIOME INFLUENCED BY ROOTS OF A
       PLANT USING  MICROPEPTIDES

<130>  BET20P4235

<160>  10

<170>  PatentIn version 3.5

<210>  1
<211>  16
<212>  PRT
<213>  Arabidopsis thaliana

<400>  1

Met Thr Trp Pro Leu Leu Ser Leu Ser Phe Leu Leu Ser Lys Tyr Val
1               5                   10                  15


<210>  2
<211>  20
<212>  PRT
<213>  Arabidopsis thaliana

<400>  2

Met Gly Leu Arg Lys Val Leu Glu Met Asn Thr Ile Phe Asp Ser Leu
1               5                   10                  15


Phe Leu Ser His
            20


<210>  3
<211>  16
<212>  PRT
<213>  Arabidopsis thaliana

<400>  3

Met Gln Asn Leu Arg Val His Val Phe Leu Ile Glu Ser Ala Arg Cys
1               5                   10                  15


<210>  4
<211>  16
<212>  PRT
<213>  Artificial Sequence

<220>
<223>  scrumbled sequence

<400>  4

Ile His Ser Gln Glu Met Leu Arg Val Cys Arg Ala Leu Phe Asn Val
1               5                   10                  15

<210>  5
<211>  19
<212>  DNA
<213>  Artificial Sequence

<220>
<223>  primer

<400>  5
gtgycagcmg ccgcggtaa                                                    19

<210>  6
<211>  20
<212>  DNA
<213>  Artificial Sequence

<220>
<223>  primer

<400>  6
ccgycaatty mtttragttt                                                   20

<210>  7
<211>  18
<212>  DNA
<213>  Artificial Sequence

<220>
<223>  primer

<220>
<221>  modified_base
<222>  (13)..(13)
<223>  I

<220>
<221>  misc_feature
<222>  (13)..(14)
<223>  n is a, c, g, or t

<220>
<221>  modified_base
<222>  (14)..(14)
<223>  I

<400>  7
gaacgcagcr aanngyga                                                     18

<210>  8
<211>  20
<212>  DNA
<213>  Artificial Sequence

<220>
<223>  primer

<400>  8
tcctccgctt attgatatgc                                                    20

<210>  9
<211>  17
<212>  DNA
<213>  Artificial Sequence

<220>
<223>  primer

<220>
<221>  misc_feature
<222>  (9)..(9)
<223>  n is a, c, g, or t

<400>  9
cctacgggng gcwgcag                                                       17

<210>  10
<211>  19
<212>  DNA
<213>  Artificial Sequence

<220>
<223>  primer

<400>  10
attaccgcgg ctgctggca                                                     19